# EUROPEAN PATENT SPECIFICATION

(11) **EP 2 021 360 B1**
(45) Date of publication and mention of the grant of the patent: **14.09.2011**
(21) Application number: 06755972.4
(22) Date of filing: 26.04.2006
(51) Int. Cl.: C07K 14/35, C12P 21/00

(54) **CYCLODIPEPTIDE SYNTHETASE AND ITS USE FOR SYNTHESIS OF CYCLO(TYR-Xaa) CYCLODIPEPTIDES**
CYCLODIPEPTIDSYNTHETASE UND IHRE VERWENDUNG ZUR SYNTHESE VON CYCLO(TYR-XAA)CYCLODIPEPTIDEN
LA SYNTHÉTASE CYCLODIPEPTIDIQUE ET SON UTILISATION POUR LA SYNTHÈSE DE CYCLODIPEPTIDES CYCLO(TYR-Xaa)

(43) Date of publication of application: 11.02.2009
(73) Proprietor: Commissariat à l'Énergie Atomique et aux Énergies Alternatives, 75015 Paris (FR); Kyowa Hakko Bio Co., Ltd, Chiyoda-ku Tokyo 100-8185 (JP); UNIVERSITE PARIS-SUD (PARIS 11), 91405 Orsay (FR); Centre National de la Recherche Scientifique, 75016 Paris Cedex 16 (FR)
(72) Inventor: BELIN, Pascal, F-91430 Igny (FR); GONDRY, Muriel, F-91470 Limours (FR); THAI, Robert, F-91620 Nozay (FR); PERNODET, Jean-Luc, F-94230 Cachan (FR); GENET, Roger, F-91470 Limours (FR)
(74) Representative: Thomas, Dean
(86) International application number: PCT/IB2006/001852
(87) International publication number: WO 2007/122447

(56) References cited:
- FR-A1- 2 841 260
- DATABASE EMBL [Online] 1 November 1996 (1996-11-01), COLE ET AL.: XP002416462 retrieved from EBI Database accession no. Q50688
- DATABASE EMBL [Online] 1 October 2003 (2003-10-01), GARNIER ET AL.: XP002416463 retrieved from EBI Database accession no. Q7TYV7
- LAUTRU S ET AL: "The Albonoursin Gene Cluster of S. noursei - Biosynthesis of Diketopiperazine Metabolites Independent of Nonribosomal Peptide Synthetases" CHEMISTRY AND BIOLOGY, CURRENT BIOLOGY, LONDON, GB, vol. 9, no. 12, December 2002 (2002-12), pages 1355-1364, XP004545193 ISSN: 1074-5521
- RHEE K-H: "Cyclic dipeptides exhibit synergistic, broad spectrum antimicrobial effects and have anti-mutagenic properties" INTERNATIONAL JOURNAL OF ANTIMICROBIAL AGENTS, AMSTERDAM, NL, vol. 24, no. 5, November 2004 (2004-11), pages 423-427, XP004617403 ISSN: 0924-8579
- GONDRY M ET AL: "Cyclic dipeptide oxidase from Streptomyces noursei: Isolation, purification and partial characterization of a novel, amino acyl alpha,beta-dehydrogenase" EUROPEAN JOURNAL OF BIOCHEMISTRY, BERLIN, DE, vol. 268, March 2001 (2001-03), pages 1712-1721, XP002242439 ISSN: 0014-2956

## Description

The present invention relates to an isolated, natural or synthetic polynucleotide and to the polypeptide encoded by said polynucleotide, that is involved in the synthesis of cyclodipeptides, to the recombinant vector comprising said polynucleotide or any substantially homologous polynucleotide, to the host cell modified with said polynucleotide or said recombinant vector and also to methods for *in vitro* and *in vivo* synthesizing cyclodipeptides, in particular cyclo(Tyr-Xaa) cyclodipeptides, wherein Xaa is any amino acid and their derivatives.

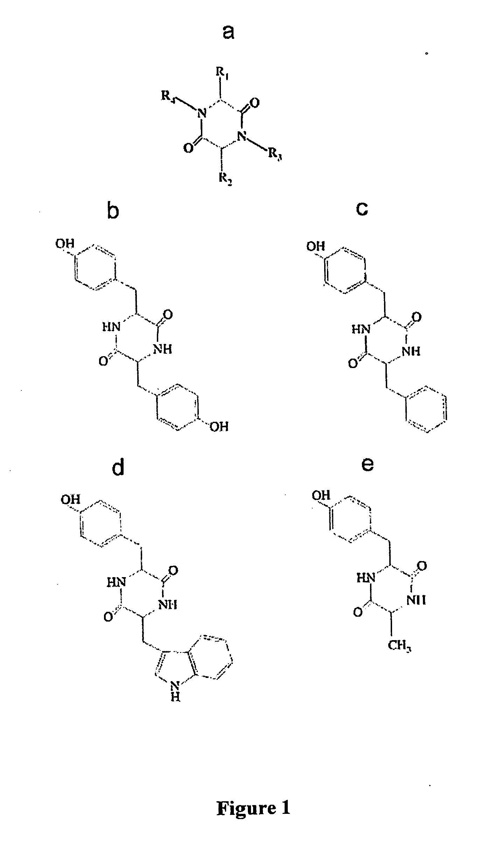

For the purposes of the present invention, the term "diketopiperazine derivatives", "DKP", "2,5-DKP", "cyclic dipeptides", cyclodipeptides or "cyclic diamino acids" is intended to mean molecules having a diketopiperazine (piperazine-2,5-dione or 2,5-dioxopiperazines) ring. In the particular case of α,β-dehydrogenated cyclodipeptide derivatives, the substituent groups R1 and R2 are α,β-unsaturated amino acyl side chains (Figure 1). Such derivatives are hereafter referred to as "Δ" derivatives.

The DKP derivatives constitute a growing family of compounds that are naturally produced by many organisms such as bacteria, yeast, filamentous fungi and lichens. Others have also been isolated from marine organisms, such as sponges and starfish. An example of these derivatives: cyclo(L-His-L-Pro), has been shown to be present in mammals.

The DKP derivatives display a very wide diversity of structures ranging from simple cyclodipeptides to much more complex structures. The simple cyclodipeptides constitute only a small fraction of the DKP derivatives, the majority of which have more complex structures in which the main ring and/or the side chains comprise many modifications: introduction of carbon-based, hydroxyl, nitro, epoxy, acetyl or methoxy groups, and also the formation of disulfide bridges or of heterocycles. The formation of a double bond between two carbons is also quite widespread. Certain derivatives, of marine origin, incorporate halogen atoms.

Useful biological properties have already been demonstrated for some of the DKP derivatives. Bicyclomycin (Bicozamine™) is an antibacterial agent used as food additive to prevent diarrhea in calve and swine (Magyar et al., J. Biol. Chem., 1999, 274, 7316-7324). Gliotoxin has immunosuppressive properties which were evaluated for the selective *ex vivo* removal of immune cells responsible for tissue rejection (Waring et al., Gen. Pharmacol., 1996, 27, 1311-1316). Several compounds such as ambewelamides, verticillin and phenylahistin exhibit antitumour activities involving various mechanisms (Chu et al., J. Antibiot. (Tokyo), 1995, 48, 1440-1445 ; Kanoh et al., J. Antibiot. (Tokyo), 1999, 52, 134-141; Williams et al., Tetrahedron Lett., 1998,39, 9579-9582).

Many others like albonoursin produced by *Streptomyces noursei*, display antimicrobial activities (Fukushima et al., J. Antibiot. (Tokyo), 1973, 26, 175-176). Cyclo(Tyr-Tyr) and cyclo(Tyr-Phe) were shown to be potential cardioactive agents: cyclo(Tyr-Tyr) being a potential cardiac stimulant and cyclo(Tyr-Phe) being a cardiac inhibitor (Kilian et al., Pharmazie 2005, 60, 305-309). These two cyclodipeptides were also tested as receptor interacting agents and the two compounds were found to exhibit significant binding to opioid receptors (Kilian et al., precited). Moreover, they were evaluated as antineoplastic agents and cyclo(Tyr-Phe) was shown to induce growth inhibition of three different cultured cell lines (Kilian et al., precited). It has been described that cyclo(ΔAla-L-Val) produced by *Pseudomonas aerugirzosa* could be involved in interbacterial communication signals (Holden et al., Mol. Microbiol. 1999, 33, 1254-1266). Other compounds are described as being involved in the virulence of pathogenic microorganisms or else as binding to iron or as having neurobiological properties (King et al., J. Agr. Food Chem., 1992, 40, 834-837; Sammes, Fortschritte der Chemie Organischer Naturstoffe, 1975, 32, 51-118; Alvarez et al., J; Antibiot., 1994, 47, 1195-1201).

Although the number of known DKPs is increasing steadily, biosynthesis pathways of these compounds are still largely unexplored, leading to little knowledge regarding their synthesis.

In several cases reported so far, the formation of DKPs occurs spontaneously from linear dipeptides for which the cis-conformation of the peptide bond is favoured by the presence of an N-alkylated amino acid or a proline residue. Such spontaneous cyclisation has also been observed in the course of non ribosomal peptide synthesis of gramicidin S and tyrocidine A in *Bacillus brevis,* due to the instability of the thioester linkage during peptide elongation on peptide synthetase megacomplexes (Schwarzer et al., Chem. Biol, 2001, 8, 997-1010). Thus, in all of the known mechanisms of spontaneous DKP formation, the primary structure of the precursor dipeptide, in particular the conformation of its peptide bond, appears to be a fundamental requirement for the formation of the DKP ring to take place and for the process to result in the production of the final DKP derivative.

However, such a spontaneous cyclisation reaction cannot account for the biosynthesis of the large majority of DKP derivatives that do not contain a proline residue or an N-alkylated residue.

Known methods for producing DKP-derivatives include chemical synthesis, extraction from natural producer organisms and also enzymatic methods:
- Chemical methods can be used for synthesizing DKP derivatives (Fischer, J. Pept. Sci., 2003, 9, 9-35) but they are considered to be disadvantageous in respect of cost and efficiency as they often necessitate the use of protected amino acyl precursors and lead to the loss of stereochemical integrity. Moreover, they are not environment-friendly methods as they use large amounts of organic solvents and the like.
- Extraction from natural producer organisms can be used but the productivity remains low because the contents of desired DKP-derivatives in natural products are often low.
- Enzymatic methods, *i.e*. methods utilizing enzymes either *in vivo* (*e.g*. culture of microorganisms expressing cyclodipeptide-synthesizing enzymes or microorganism cells isolated from the culture medium) or *in vitro* (*e.g.* purified cyclodipeptide-synthesizing enzymes) can be used. Enzymes known to produce cyclodipeptides are non-ribosomal peptide synthetases (hereinafter referred to as NRPS) (Gruenewald, et al., Appl. Environ. Microbiol., 2004, 70, 3282-3291) and AlbC which is a cyclodipeptide synthetase (CDS) (Lautru et al., Chem. Biol., 2002, 9, 1355-1364; International Application WO 2004/000879):
   the enzymatic method utilizing NRPS has already been reported to produce a specific cyclodipeptide. The two genes coding for the bimodular complex TycA/TycB1 from *Bacillus brevis* (Mootz and Marahiel, J. Bacteriol., 1997, 179, 6843-6850) were coexpressed in *Escherichia coli* and gave rise to the production of cyclo(DPhe-Pro) (Gruenewald et al., Appl. Environ. Microbiol., 2004, 70, 3282-3291). The cyclodipeptide was stable, not toxic to *E. coli* and secreted in the culture medium. However, the methods utilizing NRPS appear essentially restricted to the production of cyclodipeptides containing N-alkylated residues. Moreover, the methods utilizing NRPS are difficult to implement: NRPS being large multimodular enzyme complexes, they are not easy to manipulate both at the genetic or biochemical levels.
   the enzymatic method utilizing AlbC was also described to produce specific cyclodipeptides. The expression of AlbC from *Streptomyces noursei* by heterologous hosts *Streptomyces lividans* TK21 or *E*. *coli* led to the production of two cyclodipeptides, cyclo(Phe-Leu) and cyclo(Phe-Phe) that were secreted in the culture medium (Lautru et al., Chem. Biol., 2002, 9, 1355-1364). AlbC catalyzes the condensation of two amino acyl derivatives to form cyclodipeptides containing or not containing N-alkylated residues, by an unknown mechanism. This unambiguously shows that a specific enzyme unrelated to non ribosomal peptide synthetases can catalyze the formation of DKP derivatives: AlbC is the first example of an enzyme that is directly involved in the formation of the DKP motif.

Furthermore the obtained cyclo(Phe-Leu) cyclodipeptide may be transformed into a cyclo(α,β-dehydro-dipeptide), i.e. albonoursin, or cyclo(ΔPhe-ΔLeu), an antibiotic produced by *Streptomyces noursei,* in the presence of cyclic dipeptide oxydase (CDO) which specifically catalyzed the formation of albonoursin, in a two-step sequential reaction starting from the natural substrate cyclo(L-Phe-L-Leu) leading first to cyclo (ΔPhe-L-Leu) and finally to cyclo (ΔPhe-ΔLeu) corresponding to albonoursin (Gondry et al., Eur. J. Biochem., 2001, 268, 1712-1721). Said CDO may also transform various cyclodipeptides into α,β-dehydrodipeptides (Gondry M. et al., Eur. J. Biochem., 2001, precited).

The DKP derivatives exhibit various biological functions, making them useful entities for the discovery and development of new drugs, food additives and the like. Accordingly, it is necessary to be able to have large amounts of these compounds available.

An understanding of the pathways for the natural synthesis of the diketopiperazine derivatives could enable a reasoned genetic improvement in the producer organisms, and would open up perspectives for substituting or improving the existing processes for synthesis (via chemical or biotechnological pathways) through the optimization of production and purification yields. In addition, modification of the nature and/or of the specificity of the enzymes involved in the biosynthetic pathway for the diketopiperazine derivatives could result in the creation of novel derivatives with original molecular structures and with optimized biological properties.

The Inventors have now obtained a new cyclodipeptide synthesizing enzyme (or cyclodipeptide synthetase or CDS) family which is able to catalyze the formation of cyclo(Tyr-Xaa) cyclodipeptides, wherein Xaa is any proteinogenic or non-proteinogenic amino acid, preferably Xaa is selected among amino acid bearing an aromatic or an alkyl side chain, and even more preferably, Xaa is selected among Tyr, Phe, Trp, and Ala.

Therefore an object of the present invention is an isolated cyclodipeptide synthetase, characterized in that:
- it has the ability to produce cyclo(Tyr-Xaa) cyclodipeptides from two amino acids Tyr and Xaa, wherein Xaa is any amino acid, and
- it consists of a polypeptide selected in the group consisting of SEQ ID NO:3 and SEQ ID NO:6.

The percentages of identity and the percentages of similarity defined herein can be obtained using the BLAST program (blast2seq, default parameters) (Tatutsova and Madden, FEMS Microbiol Lett., 1999, 174, 247-250) over a comparison window consisting of the full-length of the sequence SEQ ID NO: 3 and preferably by calculating them on an overlap representing at least 90% of the length of SEQ ID NO:3, i.e. in 217 aminoacid overlap.

Another object of the present invention is an isolated polynucleotide selected from:
a) a polynucleotide encoding a cyclodipeptide synthetase selected in the group consisting of the sequences SEQ ID NO:1 and SEQ ID NO:5;
b) a complementary polynucleotide of the polynucleotide a).

Said isolated polynucleotide advantageously hybridizes to a complementary polynucleotide of the polynucleotide sequence of SEQ ID NO:1 under stringent conditions, and encodes a cyclodipeptide synthesizing enzyme having the ability to produce cyclo(Tyr-Xaa), wherein Xaa is any amino acid.

The term "hybridize(s)" as used herein refers to a process in which polynucleotides hybridize to the recited nucleic acid sequence or parts thereof. Therefore, said nucleic acid sequence may be useful as probes in Northern or Southern Blot analysis of RNA or DNA preparations, respectively, or can be used as oligonucleotide primers in PCR analysis dependent on their respective size. Preferably, said hybridizing polynucleotides comprise at least 10, more preferably at least 15 nucleotides while a hybridizing polynucleotide of the present to be used as a probe preferably comprises at least 100, more preferably at least 200, or most preferably at least 500 nucleotides.

It is well known in the art how to perform hybridization experiments with nucleic acid molecules, i.e. the person skilled in the art knows what hybridization conditions she/he has to use in accordance with the present invention. Such hybridization conditions are referred to in standard text books such as Sambrook et al., Molecular Cloning: A Laboratory Manual; Cold Spring Harbor Laboratory Press, 2nd edition 1989 and 3rd edition 2001; Gerhardt et al.; Methods for General and Molecular Bacteriology; ASM Press, 1994; Lefkovits; Immunology Methods Manual: The Comprehensive Sourcebook of Techniques; Academic Press, 1997; Golemis; Protein-Protein Interactions: A Molecular Cloning Manual; Cold Spring Harbor Laboratory Press, 2002 and other standard laboratory manuals known by the person skilled in the Art or as recited above. Preferred in accordance with the present inventions are stringent hybridization conditions.

"Stringent hybridization conditions" refer, e.g. to an overnight incubation at 42°C in a solution comprising 50% formamide, 5xSSC (750 mM NaCl, 75 mM sodium citrate), 50 mM sodium phosphate (pH 7.6), 5x Denhardt's solution, 10% dextran sulfate, and 20 µg/ml denatured, sheared salmon sperm DNA, followed e.g. by washing the filters in 0.2 x SSC at about 65°C.

Also contemplated are nucleic acid molecules that hybridize at low stringency hybridization conditions. Changes in the stringency of hybridization and signal detection are primarily accomplished through the manipulation of formamide concentration; salt conditions, or temperature. For example, lower stringency conditions include an overnight incubation at 37°C in a solution comprising 6 x SSPE (20 x SSPE = 3 mol/l NaCl; 0.2 mol/l NaH₂PO₄; 0.02 mol/l EDTA, pH 7.4), 0.5% SDS, 30% formamide, 100 µg/ml salmon sperm blocking DNA; followed by washes at 50°C with 1 x SSPE, 0.1% SDS.

In addition, to achieve even lower stringency, washes performed following stringent hybridization can be done at higher salt concentrations (e.g. 5 x SSC). It is of note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations.

An example of an isolated polynucleotide of the invention is the polynucleotide of sequence SEQ ID NO: 2 corresponding to the sequence known as Rv2275 gene, isolated from *Mycobacterium tuberculosis* (SEQ ID NO: 2 corresponds to positions from 2546883 to 2547752 of *Mycobacterium tuberculosis* H37Rv complete genome of GenBank accession number GI:57116681) and which encodes a cyclodipeptide synthetase of sequence SEQ ID NO: 4. The information available on the different databases concerns hypothetical proteins, which were not isolated and for which no function has been defined.

Another example of an isolated polynucleotide of the invention is the polynucleotide of sequence SEQ ID NO: 5 corresponding to a variant of SEQ ID NO: 2 wherein 2^{nd} codon is a GCA codon (instead of a TCA codon in SEQ ID NO:2) and which encodes a cyclodipeptide synthetase of sequence SEQ ID NO: 6 wherein 2^{nd} amino acid is Ala (instead of Ser). A further example of an isolated polynucleotide of the Invention is the polynucleotide of the sequence SEQ ID NO: 1 corresponding to the 5'-truncated sequence of SEQ ID NO: 2 starting from 49^{th} codon (TTT) wherein said 49^{th} codon is replaced with an ATG codon and 50^{th} codon is either unchanged (CAG) or replaced with a GAG codon, and which encodes a truncated form of a cyclodipeptide synthetase of sequence SEQ ID NO: 3 wherein 2^{nd} amino acid is either Gln or Glu.

According to an alternative embodiment of the invention, said polynucleotide has a polynucleotide sequence other than SEQ ID NO:2.

Isolated polynucleotides of the invention can be obtained from DNA libraries, particularly from *Mycobacterium* DNA libraries, for example from a *Mycobacterium tuberculosis* or a *Mycobacterium bovis* DNA library, using SEQ ID NO: 1 as a probe. Polynucleotides of the invention can also be obtained by means of a polymerase chain reaction (PCR) carried out on the total DNA of a *Mycobacterium,* in particular of *Mycobacterium tuberculosis* or *Mycobacterium bovis,* or can be obtained by RT-PCR carried out on the total RNA of a *Mycobacterium,* in particular of *Mycobacterium tuberculosis* or *Mycobacterium bovis.*

Another object of the present invention is a recombinant vector characterised in that it comprises a polynucleotide of the present invention as defined above.

The vector used may be any known vector of the prior art. As vectors that can be used according to the invention, mention may in particular be made of plasmids, cosmids, bacterial artificial chromosomes (BACs), integrative elements of actinobacteria, viruses or else bacteriophages.

Said vector may also comprise any regulatory sequences required for the replication of the vector and/or the expression of the polypeptide encoded by the polynucleotide (promoter, termination sites, etc.).

Another object of the invention is a modified host cell into which a polynucleotide or a recombinant vector of the invention as defined above, has been introduced.

Such a modified host cell may be any known heterologous expression system using prokaryotes or eukaryotes as hosts, and is preferably a prokaryotic cell. By way of example, mention may be made of animal or insect cells, and preferably of a microorganism and in particular a bacterium such as *Escherichia coli.*

Another object of the invention is the use of a polynucleotide or of a recombinant vector of the invention as defined above, for preparing a modified host cell as defined above.

The introduction of the polynucleotide or of the recombinant vector according to the invention into the host cell to be modified can be carried out by any known method, such as, for example, transfection, infection, fusion, electroporation, microinjection or else biolistics.

Another object of the invention is the use of a cyclodipeptide synthetase, characterized in that it has the ability to produce cyclo(Tyr-Xaa) cyclodipeptides from two amino acids Tyr and Xaa, and it consists of a polypeptide sequence selected in the group consisting of SEQ ID NO:3, 4 and 6, for producing cyclodipeptides cyclo(Tyr-Xaa), wherein Xaa is selected in the group consisting of Tyr, Phe, Trp and Ala.

According to an advantageous embodiment of said use of a cyclodipeptide synthetase of the invention as defined above, said CDS is used for producing the specific cyclodipeptides cyclo(Tyr-Tyr), cyclo(Tyr-Phe), cyclo(Tyr-Trp) and cyclo(Tyr-Ala).

In another aspect, the present invention relates to a method for the synthesis of cyclo(Tyr-Xaa) cyclodipeptides wherein Xaa is selected in the group consisting of Tyr, Phe, Trp and Ala, characterized in that it comprises:
(1) incubating two amino acids, Tyr and Xaa, under suitable conditions, with a cyclodipeptide synthetase selected in the group consisting of SEQ ID NO:3, 4 and 6, and
(2) recovering the cyclo(Tyr-Xaa) cyclodipeptides thus obtained.

In another aspect, the present invention relates to a method for the synthesis of α,β-dehydrogenated cyclo(Tyr-Xaa) cyclodipeptides, wherein Xaa is selected in the group consisting of Tyr, Phe, Trp and Ala, characterized in that it comprises:
(1') incubating two amino acids Tyr and Xaa, under the suitable conditions as defined hereabove with a cyclodipeptide synthetase selected in the group consisting of SEQ ID NO:3, 4 and 6 and a purified CDO, and
(2') recovering the α,β-dehydrogenated cyclodipeptides.

The term "suitable conditions" is preferably intended to mean the appropriate conditions (concentrations, pH, buffer, temperature, time of reaction, etc...) under which the amino acids and the cyclodipeptide synthetase of the invention are incubated to allow the synthesis of said cyclodipeptides.

An example of an appropriate concentration of amino acids and cyclodipeptide synthetase is the following: amino acids (Tyr and Xaa) are at a concentration of 0.1 mM to 100 mM, preferably of 1 mM to 10 mM; the cyclodipeptide synthetases of the invention are at a concentration of 0.1 nM to 100 µM, preferably of 1 µM to 100 µM.

An example of an appropriate buffer is 100 mM Tris-HCl containing 150 mM NaCl, 10 mM ATP, 20 mM MgCl₂ supplemented with a soluble prokaryote cell extract.

Appropriate pH is ranging between 6 and 8, appropriate temperature is ranging between 20 and 40°C, and appropriate time is ranging between 12 and 24 hours.

Therefore, according to a prefered embodiment of carrying out said method, step (1) or step (1') is performed in presence of appropriate aminoacids at a concentration between 0.1 mM to 100 mM, preferably of 1 mM to 10 mM, a cyclodipeptide synthetase according to the invention at a concentration between 0.1 nM and 100 µM, preferably of 1 µM to 100 µM, in a buffer at a pH between 6 and 8, and containing a soluble extract of prokaryote cells such as *E*. *coli* or *Streptomyces* cells which do not produce cyclodipeptide synthetase.

α, β-dehydrogenated cyclodipeptide derivatives may be obtained from the here above described cyclodipeptides as specified hereabove and according to the method described in Gondry et al. (Eur. J Biochem., 2001 precited) or in the International PCT Application WO 2004/000879.

For example, an amount of 5 10⁻³ units of CDO is added to the buffer used according to the method described above. One unit of CDO was defined as the amount catalyzing the formation of 1µmol of cyclo(Δphe-His) per min under standard assay conditions (Gondry et al., Eur. J. Biochem., 2001, 268, 4918-4927).

According to another preferred embodiment of the method of the synthesis of said cyclodipeptides or α,β-dehydrogenated derivatives thereby, a preliminary step (P) consisting in the use of a polynucleotide selected in the group consisting of SEQ ID NO:1, 2 and 5 for synthesizing cyclodipeptide synthetases, is performed before step (1) or step (1').

The methods of synthesis of cyclodipeptides and α, β-dehydrogenated derivatives thereof may be carried out in any suitable biological system, notably in a host such as, for example, a microorganism, for instance a bacterium such as *Escherichia coli* or *Streptomyces lividans,* or any known heterologous expression system using prokaryotes or eukaryotes as hosts, or even in acellular systems. According to a preferred embodiment, methods for the synthesis of cyclodipeptides and α, β-dehydrogenated derivatives thereof are carried out in a culture of the modified host cells of the invention expressing the cyclodipeptide synthetase of the invention.

Another object of the invention is the use of the modified host cell of the invention as defined above, for producing cyclodipeptides cyclo(Tyr-Xaa) selected in the group consisting of Tyr, Phe, Trp and Ala.

In another aspect, the present invention relates to a method for the synthesis of cyclo(Tyr-Xaa) cyclodipeptides, wherein Xaa is selected in the group consisting of Tyr, Phe, Trp and Ala, comprising the following steps:
(1') culturing a host cell modified by a polynucleotide encoding a cyclodipeptide synthetase selected in the group consisting of SEQ ID NO: 1, 2 and 5 in appropriate culture conditions for said host, and
(2') recovering the cyclodipeptides from the culture medium.

α, β-dehydrogenated cyclodipeptides derivatives may be obtained from the here above described cyclodipeptides as specified hereabove and according to the method described in Gondry et al. (Eur. J. Biochem., 2001, precited) or in the International PCT Application WO 2004/000879, in the following conditions:
(1') culturing a host cell modified by a polynucleotide encoding a cyclodipeptide synthetase selected in the group consisting of SEQ ID NO: 1, 2 and 5 in appropriate culture conditions for said host, and
(1") incubating the culture medium containing the cyclodipeptides obtained from step (1') with purified CDO, and
(2") recovering the α, β-dehydrogenated cyclodipeptides derivatives obtained from step (1") from the culture medium.

The conditions for using CDO are the same than those described above.

The recovering of the cyclodipeptides or of the α, β-dehydrogenated cyclodipeptides derivatives can be carried out directly from synthesis by means of liquid-phase extraction techniques or by means of precipitation, or thin-layer or liquid-phase chromatography techniques, in particular reverse-phase HPLC, or any method suitable for purifying peptides, one known to those skilled in the art.

Besides the above provisions, the invention also comprises other provisions which would emerge from the following description, which refers to examples of implementation of the invention and also to the attached drawings, in which:
- Figure 1. (a) Structure of piperazine-2,5-dione cycle. The *cis-*amide bond is in bold. (b) Structure of cyclo(Tyr-Tyr). (c) Structure of cyclo(Tyr-Phe). (d) Structure of cyclo(Tyr-Trp). (e) Structure of cyclo(Tyr-Ala).

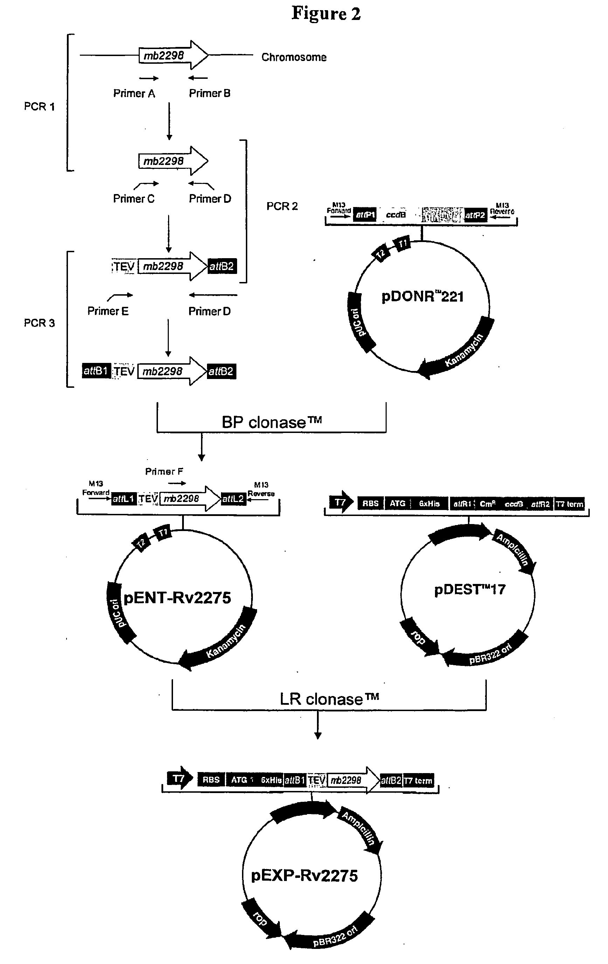

- Figure 2. The cloning strategy for the construction of the expression vector pEXP-Rv2275.

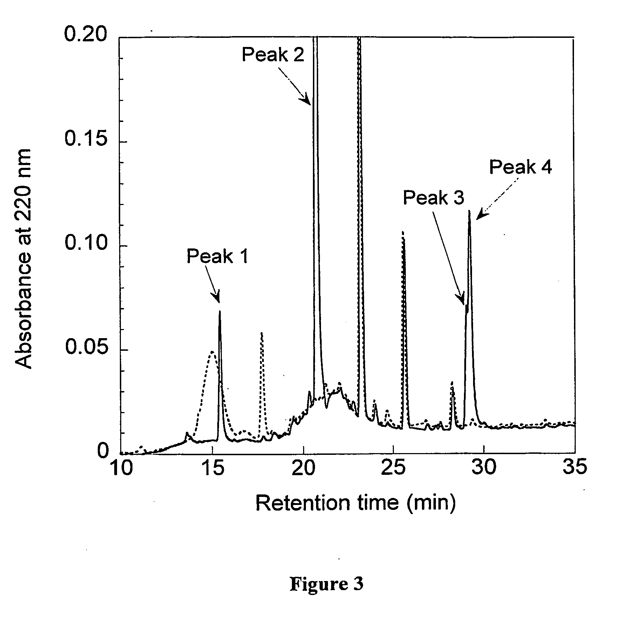

- Figure 3. HPLC analysis of the culture medium of *E*. *coli* BL21AI cells expressing the complete Rv2275 protein. Culture media of cells transformed with pEXP-Rv2275 (continuous line) and empty pQE60 (dotted line) were analyzed by RP-HPLC. Chromatograms were recorded at 220 nm.

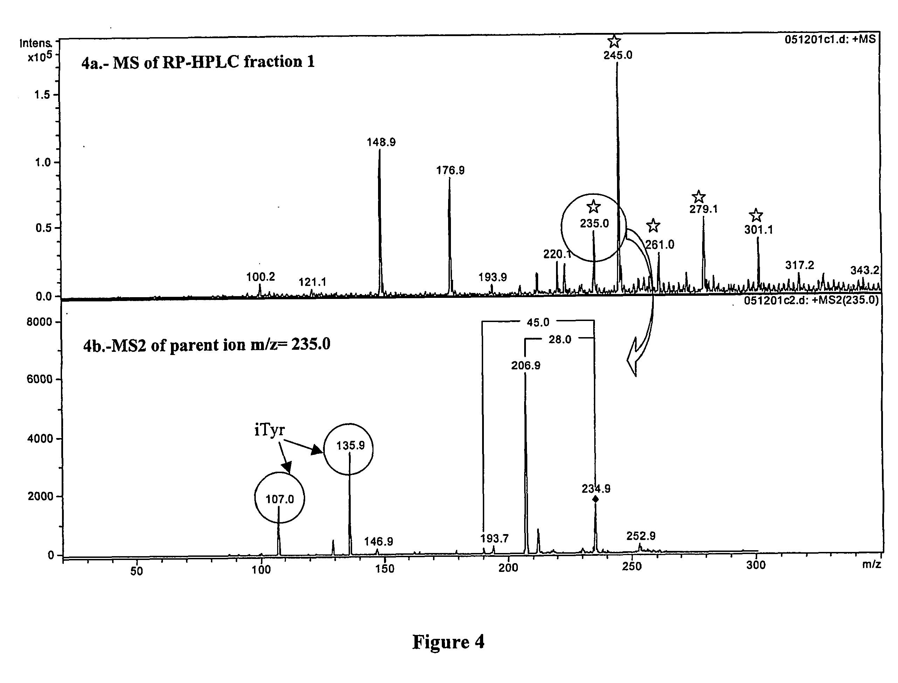

- Figure 4. MS (4a) and MSMS (4b) spectra of fraction 1 corresponding to the elution of peak 1 from the N-terminal tagged Rv2275 analysis (see Figure 3). Collected fraction 1 was directly infused into the mass spectrometer and full scan MS acquired on line (Figure 4a). m/z peaks quoted by star match to m/z of natural cyclodipeptides (Table 2) and then were selected for MSMS characterization under conditions described in Example 2. Only m/z peak at 235.0±0.1 displays a sequence of fragmentation typical to cyclodipeptides: neutral losses of 28 and 45 and production of the so-called immonium and related ion of tyrosine (hereinafter referred to as "iTyr") as shown in the daughter ion spectrum in Figure 4b.

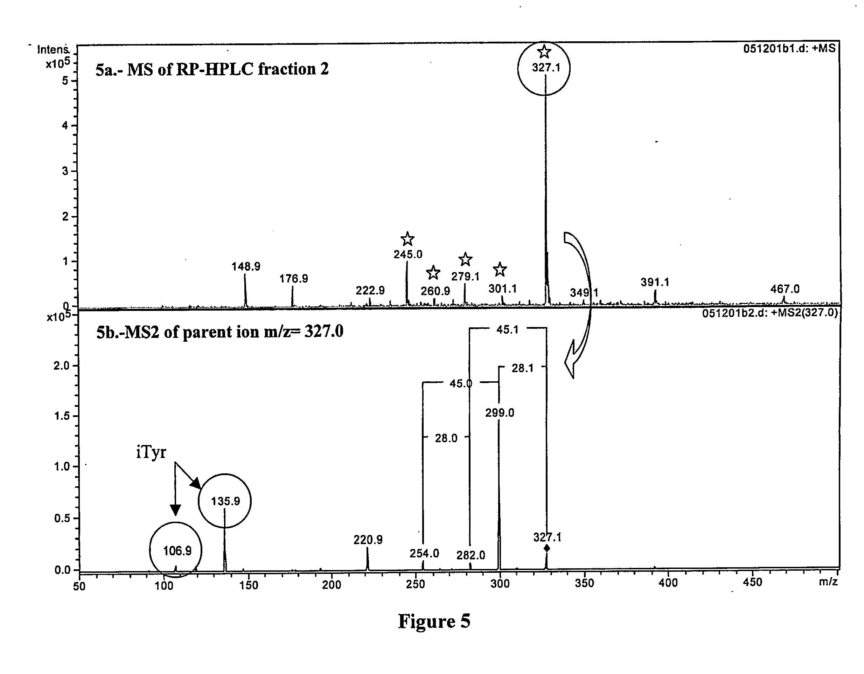

- Figure 5. MS (5a) and MSMS (5b) spectra of fraction 2 corresponding to the elution of peak 2 from the N-terminal tagged Rv2275 analysis (see Figure 3). Collected fraction 2 was directly infused into the mass spectrometer and full scan MS acquired on line (Figure 5a). Main peak with m/z at 327.0±0.1 was selected for structural characterization by MSMS fragmentation under conditions described in Example 2. Daughter ion spectrum displays a sequence of neutral losses of 28 and 45 and m/z peak at 136.0±0.1, which matches to immonium ion of tyrosine (hereinafter referred to as "iTyr")(Figure 5b).

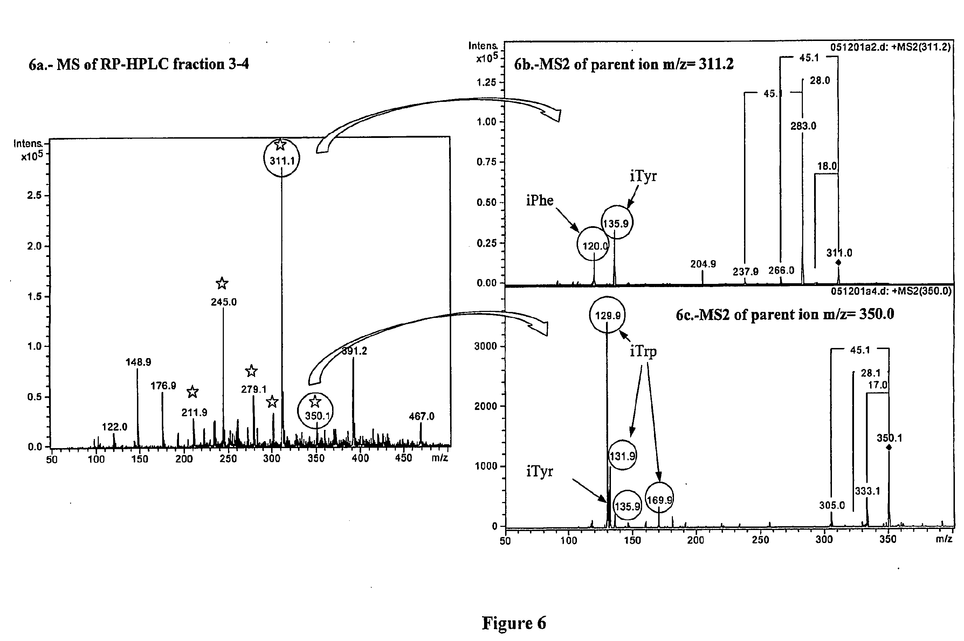

- Figure 6. MS (6a) and MSMS (6b and 6c) spectra of fractions 3-4 corresponding to the coelution of peaks 3 and 4 from the N-terminal tagged Rv2275 analysis (see Figure 3). Collected fractions 3-4 were directly infused into the mass spectrometer and full scan MS acquired on line (Figure 6a). Daughter ions spectra of the designated cyclodipeptides matched m/z peak (encircled peak in the MS spectra) were obtained under conditions described in Example 2. Immonium ion and related ion of tyrosine, phenylalanine and tryptophane are respectively referred to as "iTyr", "iPhe" and "iTrp".

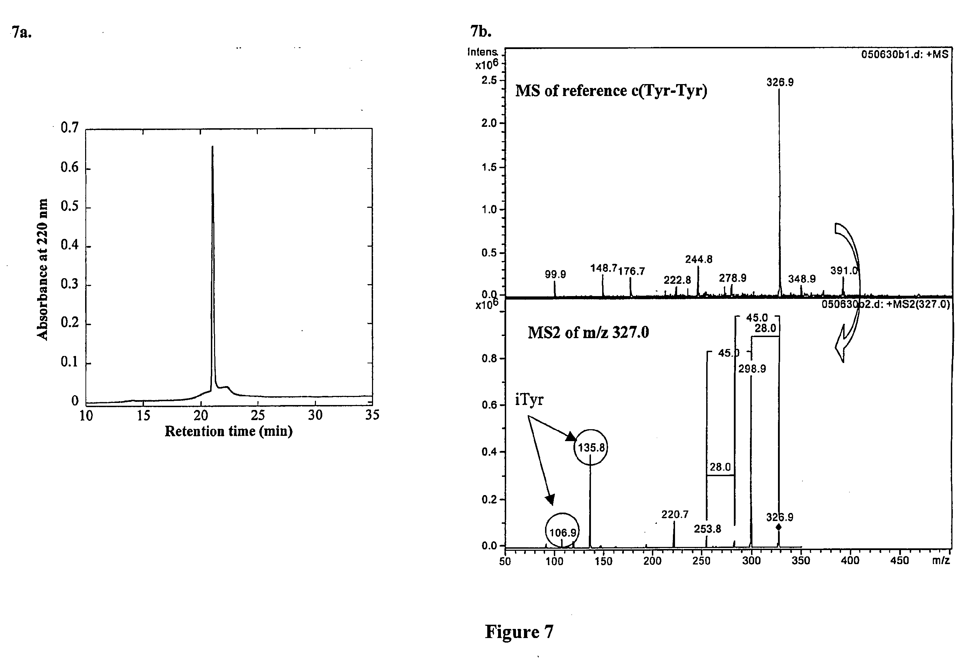

- Figure 7. HPLC analysis (7a), MS and MSMS spectra (7b) of commercial cyclo(Tyr-Tyr). The chromatogram was recorded at 220 nm. MS and daughter ions spectra of cyclo(Tyr-Tyr) at m/z 327.0±0.1 were obtained under conditions described in Experimental Methods. Immonium and related ions of tyrosine are hereinafter referred to as "iTyr".

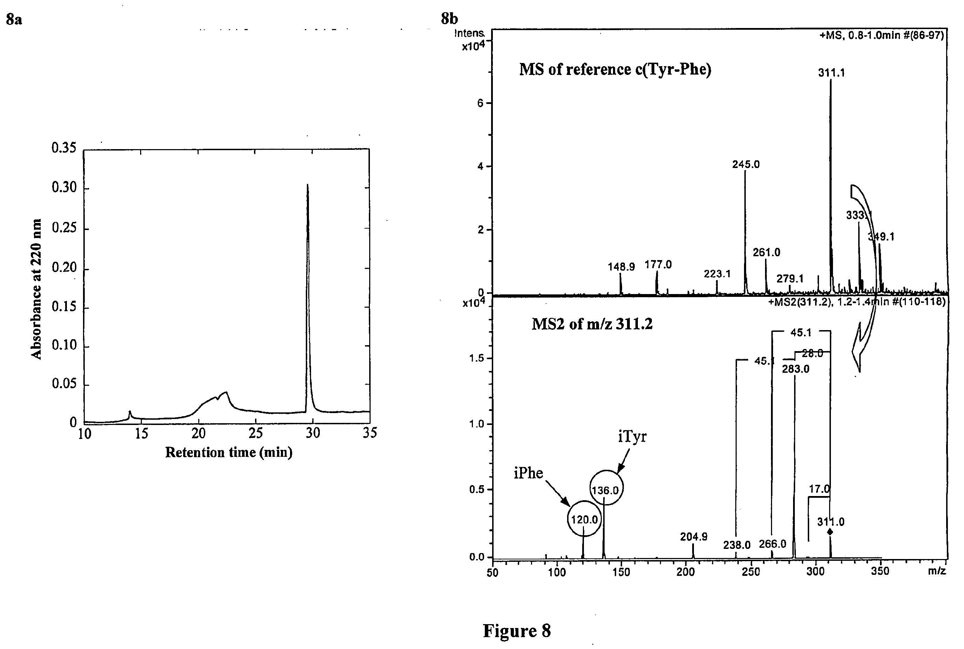

- Figure 8. HPLC analysis (8a), MS and MSMS spectra (8b) of synthesized cyclo(Tyr-Phe). The chromatogram was recorded at 220 nm. MS and daughter ions spectra of cyclo(Tyr-Phe) at m/z z0.1 were obtained under conditions described in Experimental Methods. Immonium ions of tyrosine and phenylalanine are respectively referred to as "iTyr" and "iPhe".

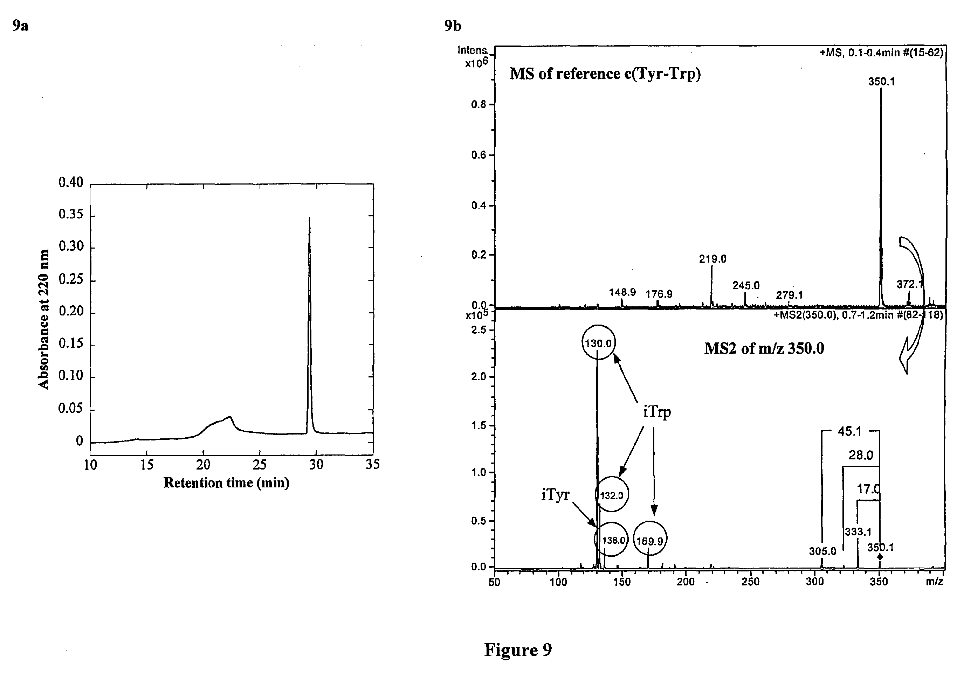

- Figure 9. HPLC analysis (9a), MS and MSMS spectra (9b) of synthesized cyclo(Tyr-Trp). The chromatogram was recorded at 220 nm. MS and daughter ions spectra of cyclo(Tyr-Trp) at m/z 350.0±0.1 were obtained under conditions described in Experimental Methods. Immonium and related ions of tyrosine and tryptophan are respectively referred to as "iTyr" and "iTrp".

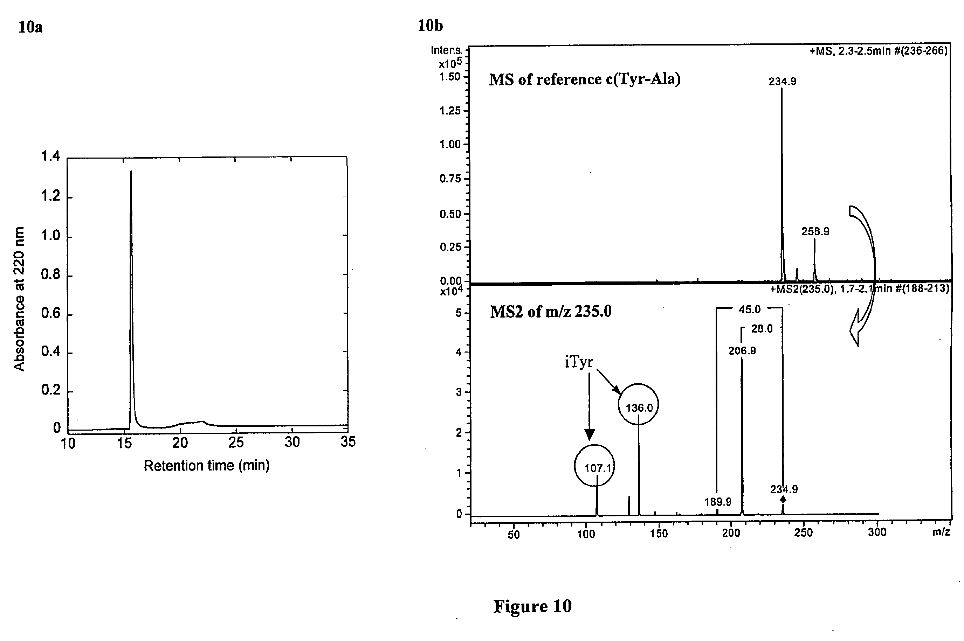

- Figure 10. HPLC analysis (10a), MS and MSMS spectra (10b) of synthesized cyclo(Tyr-Ala). The chromatogram was recorded at 220 nm. MS and daughter ions spectra of cyclo(Tyr-Ala) at m/z 235.0±0.1 were obtained under conditions described in Experimental Methods. Immonium ion of tyrosine is hereinafter referred to as "iTyr". Immonium ion of Ala is not detected.

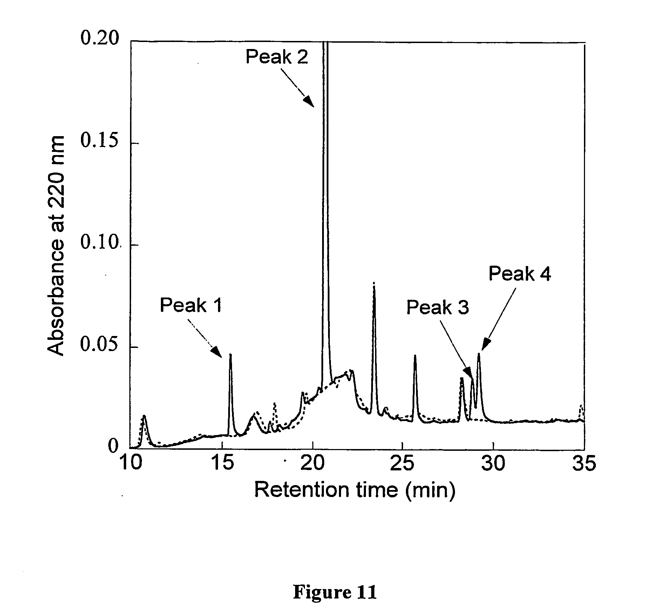

- Figure 11. HPLC analysis of the culture medium of *E*. *coli* M15[pREP4] cells expressing the truncated Rv2275 protein. Culture media of cells transformed with pQE60-Rv2275C (continuous line) and pQE60 (dotted line) were analyzed by RP-HPLC. Chromatograms were recorded at 220 nm.

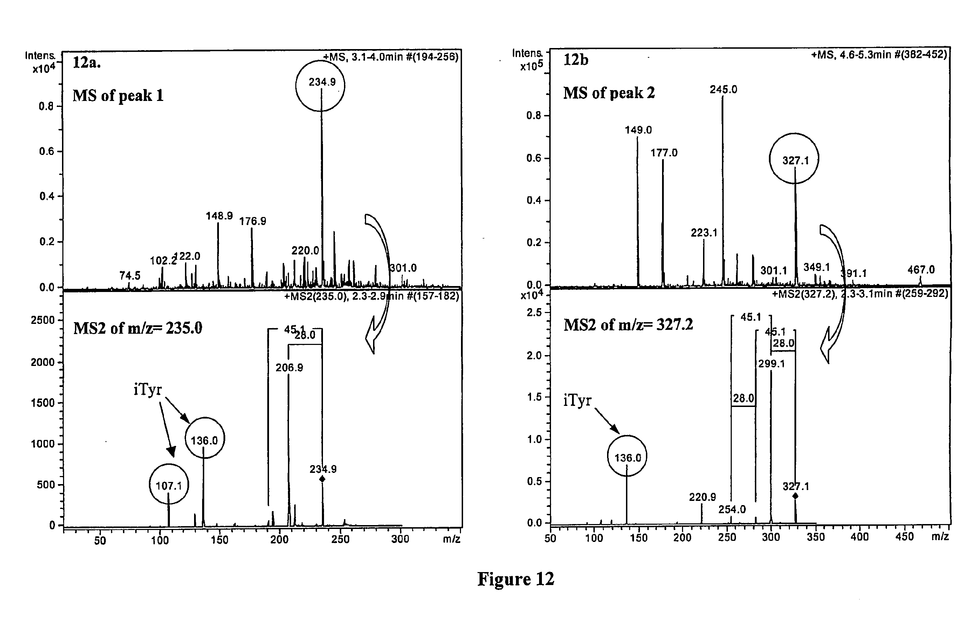

- Figure 12. MS and MSMS spectra of peak 1 (Figures 12a) and of peak 2 (Figures 12b) obtained from the truncated Rv2275 analysis (see Figure 11). Collected fractions were directly infused into the mass spectrometer and full scan MS acquired on line (top spectra). Daughter ions spectra (bottom spectra) of the designated cyclodipeptides matched m/z peak (encircled peak in the MS spectra) were obtained under conditions described in Experimental Methods. Immonium and related ions of tyrosine are hereinafter referred to as "iTyr".

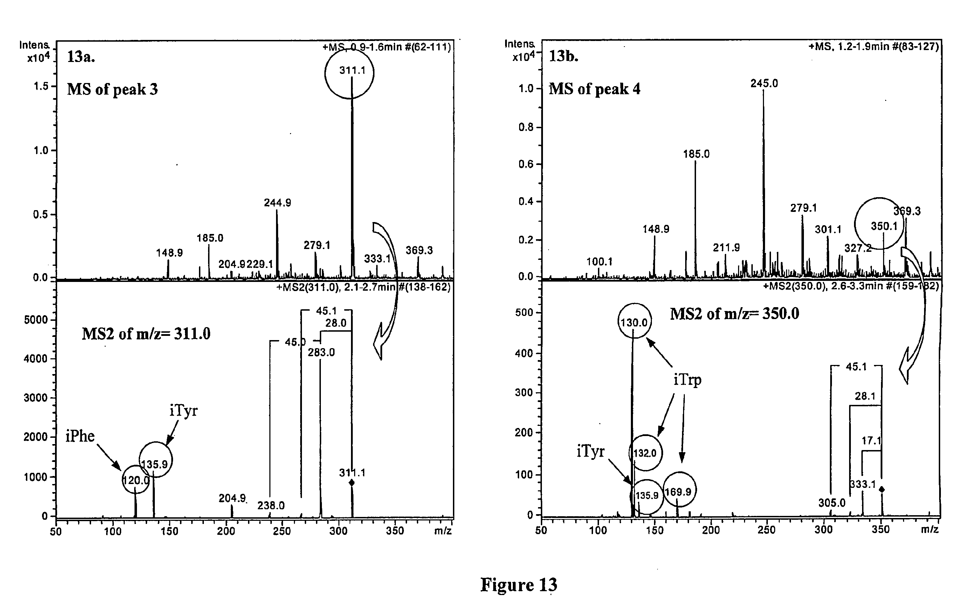

- Figure 13. MS and MSMS spectra of peak 3 (Figures 13a) and of peak 4 (Figures 13b) obtained from the truncated Rv2275 analysis (see Figure 11). Collected fractions were directly infused into the mass spectrometer and full scan MS acquired on line (top spectra). Daughter ions spectra (bottom spectra) of the designated cyclodipeptides matched m/z peak (encircled peak in the MS spectra) were obtained under conditions described in Experimental Methods. Immonium and related ions of tyrosine, phenylalanine and tryptophan are respectively referred to as "iTyr" and "iPhe" and "iTrp".

The following examples illustrate the invention but in no way limit it.

### EXAMPLE 1: CONSTRUCTION OF AN Escherichia coli EXPRESSION VECTOR ENCODING THE Rv2275 PROTEIN AS A N-TERMINAL HIS₆-TAGGED FUSION.

The expression vector encoding Rv2275 was constructed using the Gateway™ cloning technology (Invitrogen). It was designed to express Rv2275 as a cytoplasmic fusion protein carrying at its N-terminus end a (His)₆ tag, the translated sequence of the *attB* recombination site (necessary for cloning) and the TEV protease cleavage site, resulting in a N-terminal extension of 29 residues, *i.e*. MSYYHHHHHHLESTSLYKKAGFENLYFQG (SEQ ID NO: 18). As the gene encoding Rv2275 is conserved in several strains of the *Mycobacterium tuberculosis* complex (*M. tuberculosis, M. bovis*), we used for the construction of this expression vector the chromosomal DNA from *Mycobacterium bovis* BCG Pasteur that carries the *mb2298* gene (gi:31793454) 100% identical to the Rv2275 gene of *Mycobacterium tuberculosis* H37Rv (gi:15609412).

The whole cloning strategy is shown in Figure 2. First, the *attB-*flanked DNA suitable for recombinational cloning and encoding the Rv2275 protein was obtained after three successive PCR. The *mb2298* gene was amplified in the first PCR (PCR 1 in Figure 2) using *Mycobacterium bovis* BCG Pasteur genomic DNA as a template and primers A and B (Table I). The PCR conditions were one initial denaturation step at 97°C for 4 minutes followed by 25 cycles at 94°C for one minute, 54°C for one minute, 72°C for 2 minutes, and one final extension step at 72°C for 10 minutes. The reaction mixture (50 µl) comprised 1 µl of chromosomal DNA (25 ng/µl), 0.3 µl of each primer solution at 100 µM, 5 µl of 10X *Pfu* DNA polymerase buffer with MgSO₄ (provided by the *Pfu* DNA polymerase supplier), 0.1 µl of a mix of dNTPs 10 mM each and 1 µl of *Pfu* DNA polymerase (2.5 U/µl; Fermentas). The PCR product (herein after referred to as "*PCR product 1*") was then purified using the "GFX PCR DNA and Gel Band Purification" kit (Amersham Biosciences) after electrophoresis in 1% agarose gel (Sambrook et al., Molecular Cloning: A Laboratory manual, 2001, New York). The second PCR (PCR 2 in Figure 2) enabled the addition of the sequence encoding the TEV protease cleavage site to the *PCR product 1* 5'-terminus and that of the *attB2* encoding sequence to the 3'-terminus. PCR conditions were one initial denaturation step at 95°C for 5 minutes followed by 30 cycles at 95°C for 45 seconds, 50°C for 45 seconds, 72°C for 1.5 minutes, and one final extension step at 72°C for 10 minutes. The reaction mixture (50 µl) comprised 5 ng *PCR product 1*, 0.4 µM primers C and D (see Table I), 2.5 units Expand High Fidelity Enzyme mix (Roche), 1X Expand High Fidelity buffer with 1.5 mM MgCl₂ (Roche) and 200 µM each dNTP. After electrophoresis in 1% agarose gel and purification with the QIAquick Gel Extraction kit (Qiagen), the PCR product (hereinafter referred to as *"PCR products 2*") was used for the third PCR (PCR 3 in Figure 2) that enabled the addition to the *PCR product 2* 5'-terminus of the *attB1* encoding sequence. PCR was carried out as described above using 5 ng *PCR product 2* as a template and 0.4 µM primers E and D (see Table I). The resulting PCR product (hereinafter referred to as "*PCR product 3*") was purified as previously described.

Second, the *attB*-flanked *PCR product 3* was recombined with the pDONR™221 donor vector (Invitrogen) in BP clonase™ reaction to generate the entry vector pENT-Rv2275. pENT-Rv2275 was sequenced between the two-recombination sites using ABI PRISM 310 Genetic Analyzer (Applied Biosystem) and primers M13 forward, M13 reverse and F (see Table I). pENT-Rv2275 and the commercial destination vector pDEST-17 (Invitrogen) were used in LR clonase™ subcloning reaction to generate the expression vector pEXP-Rv2275 (SEQ ID NO: 19) following the supplier recommendations.

**Table I. Primers used to construct the expression vector pEXP-Rv2275.**

| Name | Corresponding sequence (5' to 3') |
|---|---|
| A | CCGTCCCTATGGTCCAAGGAAAACAATGTCATACG (SEQ ID NO: 7) |
| B | GCAAGCAATAACGGCGGGGCTCCCATCAGGGGTA (SEQ ID NO: 8) |
| C | GGCTTCGAGAATCTTTATTTTCAGGGCTCATACGTGGCTGCC (SEQ ID NO: 9) |
| D | GGGGACCACTTTGTACAAGAAAGCTGGGTCCTTATTCGGCGGGGCTC (SEQ ID NO: 10) |
| E | GGGGACAAGTTTGTACAAAAAAGCAGGCTTCGAGAATCTTTATTTTC (SEQ ID NO: 11) |
| F | TCGGCCATTCACCCAACAAT (SEQ ID NO: 12) |
| M13 Forward | GTAAACGACGGCCAG (SEQ ID NO: 13) |
| M13 Reverse | CAGGAAACAGCTATGAC (SEQ ID NO: 14) |

The recombination mixture was used for transformation of *E. coli* DH5α chemically competent cells and a positive clone was selected after analysis by colony PCR. The 50 µl reaction mix comprised a small amount of colony as a template, 200 µM each dNTP, 0.2 µM primer M13 forward and M13 reverse, 1X ThermoPol Reaction Buffer (New England Biolabs) and 1.25 unit *Taq* DNA Polymerase (New England Biolabs). The PCR conditions used were the following: one initial denaturation step at 95°C for 5 minutes followed by 30 cycles at 92°C for 30 seconds, 50°C for 30 seconds, 72°C for 2 minutes. Plasmid DNA was isolated from positive clones using the Wizard DNA Purification System (Promega) and conserved at-20°C.

### EXAMPLE 2 : EXPRESSION OF Rv2275 IN Escherichia coli CYTOPLASM LEADS TO THE SYNTHESIS OF CYCLODIPEPTIDES.

The expression of Rv2275 was performed by cultivating *E. coli* cells harboring the plasmid pEXP-Rv2275 in minimal medium. This medium contains all the elements of the M9 minimal medium (6 g/l Na₂HPO₄, 3 g/l KH₂PO₄, 0.5 g/l NaCl, 1 g/l NH₄Cl, 1mM MgSO₄, 0.1 mM CaCl₂, 1 µg/ml thiamine and 0.5% glucose or glycerol) (Sambrook *et al*., aforementioned) plus 1 ml of a vitamins solution and 2 ml of an oligoelements solution per liter of minimal medium. Vitamins solution contains 1.1 mg/l biotin, 1.1 mg/l folic acid, 110 mg/l para-aminobenzoic acid, 110 mg/l riboflavin, 220 mg/l pantothenic acid, 220 mg/l pyridoxine-HCl, 220 mg/l thiamine and 220 mg/l niacinamide in 50% ethanol. Oligoelements solution was made by diluting a FeCl₂-containing solution 50 fold in H₂O. The FeCl₂-containing solution contains for 100 ml: 8 ml concentrated HCl, 5g FeCl₂.4H₂O, 184 mg CaCl₂.2H2O, 64 mg H₃BO₃, 40 mg MnCl₂.4H₂O, 18 mg CoCl₂.6H₂O, 4 mg CuCl₂.2H₂O, 340 mg ZnCl₂, 605 mg Na₂MoO4.2H₂O.

Recombinant expression of Rv2275 from the plasmid pEXP-Rv2275 was made using *E coli* BL21AI cells (Invitrogen). 50 µl chemically competent cells were transformed with 20 ng plasmid using standard heat-shock procedure (Sambrook *et al.,* aforementioned). BL21AI cells were also transformed by pQE60 in order to make the CDS-non producing control culture. After 1 h outgrowth in SOC medium (Sambrook et al., aforementioned) at 37°C, bacteria of the two transformation mix were spread on LB plates containing 200 µg/ml ampicillin and incubated overnight at 37°C. A few colonies were picked up to inoculate M9 liquid medium supplemented with vitamins and oligoelements solutions containing 0.5% glucose and 200 µg/ml ampicillin. After overnight incubation at 37°C with shaking, 500 µl of each starter culture were used to inoculate 25 ml M9 minimal medium supplemented with vitamins and oligoelements solutions containing 0.5% glycerol and 200 µg/ml ampicillin. Bacteria were grown at 37°C until OD₆₀₀ ~ 0.5 and 0.02% L-arabinose was added. Cultures were continued at 20°C for 24 h. Cultures supernatants were collected after centrifugation at 3,000 g for 20 minutes and kept at -20°C.

### Detection of cyclodipeptide derivatives by HPLC analysis.

The formation of cyclodipeptides has been investigated by analyzing the culture supernatant of *E*. *coli* cells expressing Rv2275, as previously reported for the culture supernatant of *E*. *coli* cells expressing AlbC (Lautru et al., Chem. Biol., 2002, 9, 1355-1364).

Culture supernatants (200 µl) were acidified down to pH=3 with concentrated trifluoroacetic acid and then analyzed by HPLC. Samples were loaded onto a C18 column (4.6 x 250 mm, 5µm, 300 A, Vydac) and eluted with a linear gradient from 0% to 55% acetonitrile/deionized water with 0.1% trifluoroacetic acid for 60 min (flow-rate, 1 ml/min). The elution was monitored between 220 and 500 nm using a diode array detector.

HPLC analysis at 220 nm of the culture supernatant of *E*. *coli* cells expressing N-terminal tagged Rv2275 showed two resolved peaks, namely peak 1 and peak 2, and two poorly-resolved peaks, namely peak 3 and peak 4, that were not found in the supernatant of a culture of cells which did not express Rv2275 (empty pQ60 used) (Figure 3). These four peaks hence corresponded to products whose synthesis was directly linked to the expression of Rv2275 in *E*. *coli.* Peak 2 was the major peak and it was characterized by a retention time of 21.3 min and an absorption band centered at around 275 nm. Peak 1 was the minor peak and it was characterized by a retention time of 15.9 min and an absorption band centered at around 275 nm. Peaks 3 and 4 are not completely resolved as they displayed very close retention times. Peak 3 was characterized by a retention time of 29.6 min and an absorption band centered at around 277 nm with a shoulder at 288 nm. Peak 4 was characterized by a retention time of 29.8 min and an absorption band centered at around 275 nm. The four peaks were collected for further analysis by mass spectrometry.

### Identification of cyclodipeptide derivatives by MS and MSMS analysis.

HPLC-eluted fractions from culture supernatants (see above) were collected and analyzed by mass spectrometry using an ion trap mass analyzer Esquire HCT equipped with an orthogonal Atmospheric Pressure Interface-ElectroSpray Ionization (AP-ESI) source (Bruker Daltonik GmbH, Germany). The samples were directly infused into the mass spectrometer at a flow rate of 3 µl/min by means of a syringe pump. Nitrogen served as the drying and nebulizing gas while helium gas was introduced into the ion trap for efficient trapping and cooling of the ions generated by the ESI as well as for fragmentation processes. Ionization was carried out in positive mode with a nebulizing gas set at 9 psi, a drying gas set at 5 µl/min and a drying temperature set at 300°C. Ionization and mass analyses conditions (capillary high voltage, skimmer and capillary exit voltages and ions transfer parameters) were set for an optimal detection of compounds in the range of cyclodipeptides masses between 100 and 400 m/z. For structural characterization by mass fragmentations, an isolation width of 1 mass unit was used for isolating the parent ion. Fragmentation amplitude was tuned until at least 90% of the isolated precursor ion was fragmented. Full scan MS and MSMS spectra were acquired using EsquireControl software and all data were processed using DataAnalysis software.

Commercial cyclodipeptides (Sigma and Bachem) or chemically-synthesized cyclodipeptides (as described in Jeedigunta et al., Tetrahedron, 2000, 56, 3303-3307) were used as standard for mass and HPLC analyses.

MS spectra of the eluted fractions corresponding to the N-terminal tagged Rv2275, hereinafter referred to as "fraction 1" for the elution of peak 1, "fraction 2" for the elution of peak 2 and "fraction 3-4" for the elution of both peaks 3 and 4 were respectively shown in Figures 4a, 5a & 6a. These spectra showed heterogeneous masses and rather low intensity m/z peaks. This might be due to the difficulty of the cyclodipeptides potentially produced by Rv2275 to be ionized by electrospray ionisation. Starting from MS spectra, we compared all significant m/z values (Signal/Noise ratio > 5) to expected mass values of natural cyclodipeptides (quoted in Table II).

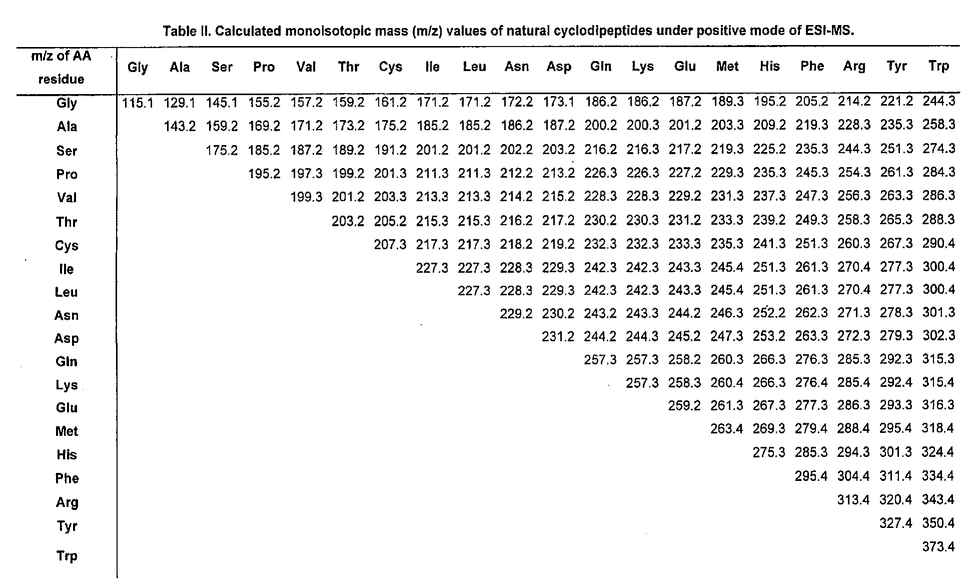

In a second step, MSMS experiments were performed in order to elucidate the chemical structure of the compounds whose m/z values matched to that of cyclodipeptides.

As already experimented on different commercial or home-made synthetic cyclodipeptides and also observed on cyclodipeptides daughter ions spectra published elsewhere (Chen et al., Eur. Food Research technology, 2004, 218, 589-597; Stark et al., J. Agric. Food Chem., 2005, 53, 7222-7231), cyclodipeptide derivatives are fragmented following a characteristic pattern: (i) a sequence of neutral losses which results from cleavages of the diketopiperazine ring on either sides of the carbonyl group (loss of 28 uma corresponding to the departure of C=O group) or of the amido group (loss of 45 corresponding to CONH₃) and (ii) the presence of m/z peaks of the so-called immonium ions and of their related ions (Roepstorff et al., Biomed. Mass Spectrom., 1984, 11, 601; Johnson et al., Anal. Chem., 1987, 59, 2621-2625) which enable to identify aminoacyl residues (Table III).

**Table III. Immonium and related ion masses m/z used for the identification of the cyclodipeptides according to Falick, A. M. et al., J. Am. Soc. Mass Spectrom., 1993, 4, 882-893 and Papayannopoulos, I. A., Mass Spectrom. Rev., 1995, 14, 49-73.**

| **Residue** | **Immonium ion*** | **Related ions*** |
|---|---|---|
| **Gly** | 30 | |
| **Ala** | 44 | |
| **Ser** | 60 | |
| **Pro** | **70** | |
| **Val** | 72 | 41,55, 69 |
| **Thr** | 74 | |
| **Cys** | *76* | |
| **Ile** | **86** | 44, *72* |
| **Leu** | **86** | 44*, 72* |
| **Asn** | *87* | *70* |
| **Asp** | *88* | *70* |
| **Gln** | 101 | 56, 84, *129* |
| **Lys** | *101* | 70, 84, 112, 129 |
| **Glu** | *102* | |
| **Met** | *104* | 61 |
| **His** | **110** | *82, 121, 123, 138,* 166 |
| **Phe** | **120** | *91* |
| **Arg** | *129* | 59*,* 70*, 73,* 87*,* 100*,* 112 |
| **Tyr** | **136** | *91, 107* |
| **Trp** | **159** | 77, 117, **130**, 132, **170**, **171** |

| | | |
|---|---|---|
| * Bold face indicates strong signals, italic indicates weak. | | |

The matched cyclodipeptide m/z peaks (marked with a star in Figures 4a, 5a & 6a) were then subjected individually to MSMS fragmentations and all the obtained daughter ions spectra were screened for cyclodipeptide fragmentation pattern.

MS analysis of fraction 1 showed five m/z peaks whose m/z value matches to a natural cyclodipeptide (Figure 4a). However, only one (235.0±0.1) after MSMS fragmentation gave rise to a typical pattern of cyclodipeptide showing neutral losses of 28 and 45 and the appearance of m/z peaks corresponding to immonium (136±0.1) and related ion (107±0.1) of tyrosine (Figure 4b). According to masses shown in Table II, the only cyclodipeptide containing a tyrosyl residue with a m/z of 235 is the cyclo(Tyr-Ala). Hence, the compound eluted at a retention time of 15.9 min was shown to be cyclo(Tyr-Ala).

MS analysis of fraction 2 showed five m/z peaks whose m/z value matches to a natural cyclodipeptide (Figure 5a). However, only the major peak at 327.1±0.1 presented a fragmentation pattern typical of a cyclodipeptide (Figure 5b). Compared to m/z values of natural cyclodipeptides of Table 2, this m/z matches to cyclo(Tyr-Tyr). In addition, daughter ions spectrum of this m/z 327.1±0.1 showed the presence of immonium ion of tyrosine (Figure 5b). This result indicates that Rv2275 product eluted in the fraction 2 at 21.3 min is cyclo(Tyr-Tyr).

MS analysis of fraction 3-4 showed two peaks both matching expected m/z values for cyclodipeptides and presenting a fragmentation pattern typical of a cyclodipeptide (Figures 6): a peak with a m/z =311.1±0.1 and a smaller one with a m/z = 350.0±0.1 (Figure 6a). Structural characterization by MSMS fragmentation identified the m/z peak at 311.1±0.1 as cyclo(Tyr-Phe) in reference to detected immonium ions of tyrosine (m/z = 136.0±0.1) and of phenylalanine (m/z = 120.0±0.1) (Figure 6b). MSMS analysis of the tiny m/z peak at 350.0±0.1 produced immonium ion of tyrosine (m/z = 136.0±0.1) and three of the immonium related ions of tryptophan (m/z = 130.0±0.1, 132.0±0.1 and 170.0±0.1) (Figure 6c). Peak with a m/z at 350.0±0.1 was then attributed to cyclo(Tyr-Trp).

### EXAMPLE 3 : EXPRESSION OF Rv2275 IN Escherichia coli CYTOPLASM LEADS TO THE SYNTHESIS OF CYCLO(TYR-Xaa).

The previously presented MS analyses strongly suggest that the compounds identified in culture supernatants of *E. coli* cells producing Rv2275 were cyclo(Tyr-Ala), cyclo(Tyr-Tyr), cyclo(Tyr-Phe) and cyclo(Tyr-Trp). To confirm these identifications, the commercial cyclo(Tyr-Tyr) and cyclo(Tyr-Trp) and the chemically-synthesized cyclo(Tyr-Phe) and cyclo(Tyr-Ala) were used as references for both HPLC analyses and MSMS experiments. All of these standard cyclodipeptides display the same chromatographic and mass features detected above. Indeed, the retention time of the reference cyclo(Tyr-Tyr) (see Figure 7a) is identical to that of the peak 2 of the culture supernatant (Figure 3). Second, the reference cyclo(Tyr-Tyr) was submitted to MS and MSMS analysis and the resulting fragmentation pattern (Figure 7b) was found similar to that obtained in Figure 5b. Standard cyclo(Tyr-Phe) elutes at a retention time identical to that obtained for the poorly-resolved peak 4 (Figure 8a compared to Figure 3). The MS and MSMS features of this standard cyclodipeptide (Figure 8b) are identical to that obtained for peak 4 (Figure 6b). In the same way, RP-HPLC and MS analyses on standard cyclo(Tyr-Trp) (Figures 9a and 9b) and cyclo(Tyr-Ala) (Figures 10a and 10b) display the same RP-HPLC retention times and fragmentation patterns than that of the metabolites detected in the culture supernatant of pEXP-Rv2275.

Definitely, we showed that expression of Rv2275 in *E*. *coli* leads to the synthesis of cyclo(Tyr-Tyr), cyclo(Tyr-Ala), cyclo(Tyr-Phe) and cyclo(Tyr-Trp) found in the culture medium, demonstrating that Rv2275 is a cyclo(Tyr-Xaa)-synthesizing enzyme that can be produced in an active form in *E*. *coli.*

### EXAMPLE 4 : CONSTRUCTION OF AN Escherichia coli EXPRESSION VECTOR ENCODING A TRUNCATED Rv2275 PROTEIN (SEQ ID NO: 3) AS A C-TERMINAL HIS₆-TAGGED FUSION

Rv2275 is 289 amino acids long, compared to 239 for AlbC. The alignment of both proteins shows that in Rv2275 there is an N-terminal extension of 48 amino acids, which has no equivalent in AlbC. To address the question of the dispensability of several amino acids, two constructions were made to express two different C-terminal His₆-tagged versions of Rv2275 in the cloning vector pQE60 (Qiagen).

In the first construction, named plasmid pQE60-Rv2275L, the complete coding sequence of Rv2275 is present and in the second construction, named plasmid pQE60-Rv2275C, a truncated coding sequence lacking the first 48 codons is present.

### Construction of the plasmid pQE60-Rv2275L

A DNA fragment carrying the complete sequence encoding Rv2275 was obtained after PCR amplification using pEXP-Rv2275 as template, Taq DNA polymerase (Pharmacia) and the following primers under conditions recommended by the manufacturer:
- primer KRVLF (5'-CGGCCATGGCATACGTGGCTGCCGAACCAGGC-3', *Nco*I site underlined) (SEQ ID NO: 15), and
- primer KRVR (5'-GGCAGATCTTTCGGCGGGGCTCCCATCAGG-3', *Bgl*II site underlined) (SEQ ID NO: 16).

Two restriction sites were thus introduced: a *Nco*I site upstream of the coding sequence and a *Bgl*II site downstream. The introduction of the *Nco*I site was accompanied by the replacement of the second codon TCA by GCA (see SEQ ID NO: 5 wherein K is G), leading to the replacement of the second amino acid serine by alanine in the corresponding protein (see SEQ ID NO: 6 wherein the amino acid at location 2 stands for Ala). This DNA fragment was cloned as a *Nco*I*-Bgl*II fragment into the vector pQE60 digested by *Nco*I and *Bam*HI, yielding the plasmid pQE60-Rv2275L (SEQ ID NO: 20). The DNA sequence of the insert was confirmed by sequencing.

### Construction of the plasmid pQE60-Rv2275C

A DNA fragment carrying the sequence encoding the truncated version of Rv2275 was obtained after PCR amplification using pEXP-Rv2275 as template, Taq DNA polymerase (Pharmacia) and the following primers under conditions recommended by the manufacturer:
- primer KRVCF (5'-CGGCCATGGAGCTAGGCAGGCGCATTCCGGAAGC-3', *Nco*I site underlined) (SEQ ID NO: 17), and
- primer KRVR (5'-GGCAGATCTTTCGGCGGGGCTCCCATCAGG-3', *Bgl*II site underlined) (SEQ ID NO: 16).

Two restriction sites were thus introduced: a *Nco*I site upstream of the coding sequence and a *Bgl*II site downstream. In the fragment obtained by PCR amplification an ATG initiation codon was introduced at a position corresponding to the 49^{th} codon (TTT) in the original *Rv2275* sequence. The introduction of the *Nco*I site was also accompanied by the replacement of the codon CAG (50^{th} codon in the original sequence) by GAG (second codon in the truncated sequence) (see SEQ ID NO: 1 wherein S is G), leading to the replacement of a glutamine by glutamic acid in the corresponding protein (see SEQ ID NO: 3 wherein the amino acid at location stands for Glu). This DNA fragment was cloned as a *Nco*I*-Bgl*II fragment into the vector pQE60 digested by *Nco*I and *Bam*HI, yielding the plasmid pQE60-Rv2275C (SEQ ID NO: 21). The DNA sequence of the insert was confirmed by sequencing.

### Expression of the complete and truncated Rv2275 protein for synthesis of cyclodipeptides

The expressions of the complete and truncated versions of Rv2275 were performed by cultivating *E. coli* cells harboring the plasmid pQE60-Rv2275L or pQE60-Rv2275C, as described in Example 2.

Expressions of C-terminal tagged Rv2275 from plasmids pQE60-Rv2275L and pQE60-Rv2275C were made by using *E. coli* strain M15 containing the pREP4 plasmid (hereinafter referred as to "M15pREP4") (Qiagen). 50 µl chemically competent cells were transformed with 20 ng plasmid pQE60-Rv2275L, pQE60-Rv2275C and pQE60 using standard heat-shock procedure (Sambrook et al., aforementioned). After 1 h outgrowth in SOC medium, transformation mixture was spread on LB plates containing 0.5% glucose, 100 µg/ml ampicillin and 30 µg/ml kanamycin and incubated overnight at 37°C. A few colonies were picked up to inoculate M9 liquid medium supplemented with vitamins and oligoelements solutions containing 0.5% glucose, 100 µg/ml ampicillin and 30 µg/ml kanamycin. After 24 h growth at 37°C with 200 rpm on a rotary shaker, 25 ml preheated minimal medium supplemented with vitamins and oligoelements solutions containing 0.5% glycerol, 100 µg/ml ampicillin and 30 µg/ml kanamycin were inoculated with 500 µl starter culture in minimal medium. Bacterial cultures were incubated at 37°C with 200 rpm rotary shaking and absorbance at 600 nm (OD₆₀₀) was followed during the growth. When OD₆₀₀ reached 0.5, 1 mM IPTG was added and cultures were incubated with 200 rpm rotary shaking at 20°C for 20 hours. Bacterial cultures were then centrifuged 20 min. at 3,000 g and supernatant was saved for cyclodipeptide production analysis.

*E. coli* M15pREP4 cells harbouring the plasmid pQE60-Rv2275L were cultivated and their ability to synthesize cyclo(Tyr-Tyr), cyclo(Tyr-Ala), cyclo(Tyr-Phe) and cyclo(Tyr-Trp) was evaluated by HPLC as previously described. The chomatogram at 220 nm was found similar to that obtained with supernatant of *E*. *coli* cells harbouring the plasmid pEXP-Rv2275 (data not shown).

*E. coli* M15pREP4 cells harbouring the plasmid pQE60-Rv2275C were cultivated and their ability to synthesize cyclo(Tyr-Tyr), cyclo(Tyr-Ala), cyclo(Tyr-Phe) and cyclo(Tyr-Trp) was evaluated by HPLC as previously described (Figure 11). The chomatogram at 220 nm was found similar to that obtained with supernatant of *E*. *coli* cells harbouring the plasmid pEXP-Rv2275 (compare chromatograms in Figure 3 and Figure 11): expression of the truncated version of Rv2275 led to the presence of four peaks (quoted peak 1, 2, 3 and 4 in 11) that display respectively retention times and spectral characteristics similar to those of peaks 1, 2, 3 and 4 previously obtained with full length Rv2275 (Figure 3). As revealed by MS and MSMS analysis (Figures 12 & 13), peaks 1', 2', 3' and 4', were respectively identified as cyclo(Tyr-Ala), cyclo(Tyr-Tyr), cyclo(Tyr-Trp) and cyclo(Tyr-Phe). Therefore the N-terminal extension of Rv2275 is dispensable for the cyclo(Tyr-Xaa) synthesizing activity.

### EXAMPLE 5: IN VITRO PRODUCTION OF CYCLO(TYR-TYR) BY THE PURIFIED Rv2275 PROTEIN.

### Production of the purified Rv2275 protein

Bacteria culture for production of the Rv2275 protein was performed as already described in Example 2, except that minimal medium was replaced by LB medium (Sambrook et al., aforementioned). After induction with 0.02% arabinose, the culture was continued at 20°C for 12h. The bacterial cells were harvested by centrifugation at 4,000 g for 20 min and frozen at -80°C. Then, bacterial cells were thawed and resuspended in 1.5 ml of an extraction buffer composed of 100 mM Tris-HCl pH 8, 150 mM NaCl and 5% glycerol. Cells were broken using an Eaton press and centrifuged at 20,000 g and 4°C for 20 min. The resulting supernatant containing the soluble proteins was loaded onto a Ni²⁺-column (HisTrap HP from Amersham) equilibrated with a buffer composed of 100 mM Tris-HCl pH 8, 150 mM NaCl. The column was washed with the same buffer and submitted to a linear gradient of imidazole (from 0 to 1 M imidazole at pH 8). The Rv2275 protein was eluted at around 250 mM imidazole. The purified Rv2275 protein was then washed (to eliminate imidazole) and concentrated using a Vivaspin concentrator (Vivascience).

### Preparation of the soluble cell extract used for supplementation

Bacteria transformed with the empty vector pQE60 (Qiagen) were cultivated and broken as previously described. The broken cells were centrifuged at 20,000 g and 4°C for 20 min. The resulting supernatant corresponds to a soluble extract of *E*. *coli* cells, which does not contain cyclodipeptide synthetase.

### In vitro production of cyclo(Tyr-Tyr)

A 215 µl-reaction mixture comprising 5.5 mM Tyr, 10 mM ATP, 20 mM MgCl₂ and 25 µM of the purified Rv2275 protein was supplemented with 115 µl of the previously described soluble cell extract. This mixture was incubated at 30°C for 12h. The reaction was stopped by adding TFA and submitted to a centrifugation at 20,000 g for 20 min. The supernatant was then analyzed by HPLC and HPLC-eluted fractions were characterized by mass spectrometry as described in Example 2. As a control, the same experiment was performed under similar conditions except that the purified Rv2275 was omitted.

The results clearly showed that the incubated mixture comprising the Rv2275 protein contains cyclo(Tyr-Tyr) (an HPLC-eluted fraction at a retention time of 21.3 min with mass characteristics similar to that shown in Figure 5) whereas the incubated mixture devoid of the Rv2275 protein contains no cyclodipeptide. This demonstrates that the formation of cyclo(Tyr-Tyr) can be performed *in vitro* with a purified cyclo(Tyr-Xaa) synthetase.

The procedure described for the cyclo(Tyr-Tyr) cyclodipeptide can be applied to cyclo(Tyr-Phe), cyclo(Tyr-Trp) and cyclo(Tyr-Ala).

## Claims

1. An isolated cyclodipeptide synthetase, **characterized in that**:
- it has the ability to produce cyclo(Tyr-Xaa) cyclodipeptide from two amino acids Tyr and Baa, wherein Xaa is any amino acid, and
- it consists of a polypeptide selected in the group consisting of SEQ ID NO:3 and SEQ ID NO:6.

2. An isolated polynucleotide, **characterized in that** it is selected from:
a) a polynucleotide encoding a cyclodipeptide synthetase selected in the group consisting of the sequences SEQ ID NO:1 and SEQ ID NO:5;
b) a complementary polynucleotide of the polynucleotide a).

3. A recombinant vector comprising a polynucleotide as defined in claim 2, or a plasmid comprising said polynucleotide.

4. A host cell modified by a polynucleotide as defined in claim 2 or by a recombinant vector as defined in claim 3.

5. The host cell according to claim 4, selected in the group consisting of a prokaryotic cell, preferably a bacterium.

6. Use of a cyclodipeptide synthetase, **characterized in that** it has the ability to produce cyclo(Tyr-Xaa) cyclodipeptides from two amino acids Tyr and Xaa, and it consists of a polypeptide sequence selected in the group consisting of SEQ ID NO:3, 4 and 6, for producing cyclodipeptides cyclo(Tyr-Xaa), wherein Xaa is selected in the group consisting of Tyr, Phe, Trp and Ala.

7. Use of a host cell modified by a polynucleotide encoding a cyclodipeptide synthetase as defined in claim 6, for producing cyclodipeptides cyclo(Tyr-Xaa), wherein Baa is selected in the group consisting of Tyr, Phe, Trp and Ala.

8. A method for the synthesis of cyclo(Tyr-Xaa) cyclodipeptides, wherein Xaa is selected in the group consisting of Tyr, Phe, Trp and Ala, **characterized in that** it comprises:
(1) incubating two amino acids Tyr and Xaa, under suitable conditions, with a cyclodipeptide synthetase selected in the group consisting of SEQ ID NO:3, 4 and 6, and
(2) recovering the cyclo(Tyr-Xaa) cyclodipeptides thus obtained.

9. A method for the synthesis of α,β-dehydrogenated cyclo(Tyr-Xaa) cyclodipeptides, wherein Xaa is selected in the group consisting of Tyr,Phe, Trp and Ala, **characterized in that** it comprises:
(1') incubating two amino acids Tyr and Xaa, under suitable conditions with a cyclodipeptide synthetase selected in the group consisting of SEQ ID NO:3, 4 and 6 and a purified CDO, and
(2') recovering the α,β-dehydrogenated cyclodipeptides.

10. The method according to claim 8 or claim 9, wherein step (1) or step (1') is performed in presence of appropriate aminoacids at a concentration between 0.1 mM to 100 mM, preferably of 1 mM to 10 mM, a cyclodipeptide synthetase at a concentration between 0.1 nM and 100 µM, preferably of 1 µM to 100 µM, in a buffer at a pH between 6 and 8, and containing a soluble extract of prokaryote cells such as *E. coli* or *Streptomyces* cells which do not produce cyclodipeptide synthetase.

11. The method according to any one of claims 8 to 10, **characterized in that** it further comprises a preliminary step (P) consisting in the use of a polynucleotide selected in the group consisting of (SEQ ID NO:1, 2 and 5, for synthesizing a cyclodipeptide synthetase, which is performed before step (1) or step (1').

12. A method for the synthesis of cyclo(Tyr-Xaa) cyclodipeptides, wherein Xaa is selected in the group consisting of Tyr, Phe, Trp and Ala, comprising the following steps:
(1') culturing a host cell modified by a polynucleotide encoding a cyclodipeptide synthetase selected in the group consisting of SEQ ID NO:1,2 and 5 in appropriate culture conditions for said host, and
(2') recovering the cyclodipeptides from the culture medium.

13. A method for the synthetis α,β-dehydrogenated eyclo(Tyr-Xaa) cyclodipeptides, wherein Xaa is selected in the group consisting of Tyr, Phe, Trp and Ala, comprising the following steps:
(1') culturing a host cell modified by a polynucleotide encoding a cyclodipeptide synthetase selected in the group consisting of SEQ ID NO: 1, 2 and 5 in appropriate culture conditions for said host,
(1") incubating the cyclo(Tyr-Xaa) cyclodipeptide obtained from step (1') with a purified CDO, and
(2') recovering the cyclodipeptides from the culture medium.

## Patentansprüche

1. Isolierte Cyclodipeptidsynthetase, **dadurch gekennzeichnet, dass**:
- sie die Fähigkeit, Cyclo(Tyr-Xaa) Cyclodipeptide aus zwei Aminosäuren Tyr und Xaa zu produzieren, besitzt, wobei Xaa jegliche Aminosäure ist und
- sie aus einem Polypeptid besteht, welches aus der aus SEQ ID NO: 3 und SEQ ID NO: 6 bestehenden Gruppe ausgewählt ist.

2. Isoliertes Polynucleotid, **dadurch gekennzeichnet, dass** es ausgewählt ist aus:
a) einem Polynucleotid, welches eine Cyclodipeptidsynthetase codiert, das aus der Gruppe bestehend aus den Sequenzen SEQ ID NO: 1 und SEQ ID NO: 5 ausgewählt ist;
b) einem komplementären Polynucleotid des Polynucleotids a).

3. Rekombinanter Vektor, welcher ein Polynucleotid, wie definiert in Anspruch 2, umfasst oder ein Plasmid, welches das Polynucleotid umfasst.

4. Wirtszelle, die durch ein Polynucleotid, wie definiert in Anspruch 2, oder durch einen rekombinanten Vektor, wie definiert in Anspruch 3, modifiziert ist.

5. Wirtszelle gemäß Anspruch 4, welche aus der Gruppe, bestehend aus einer prokaryontischen Zelle, bevorzugt einem Bakterium, ausgewählt ist.

6. Verwendung einer Cyclodipeptidsynthetase, **dadurch gekennzeichnet, dass** sie die Fähigkeit besitzt, Cyclo(Tyr-Xaa)-cyclodipeptide aus zwei Aminosäuren Tyr und Xaa zu produzieren und die aus einer Polypeptidsequenz besteht, welche aus der Gruppe ausgewählt ist, die aus SEQ ID NO: 3, 4 und 6 besteht, zur Herstellung von Cyclodipeptiden Cyclo(Tyr-Xaa), wobei Xaa innerhalb der Gruppe ausgewählt wird, die aus Tyr, Phe, Trp und Ala besteht.

7. Verwendung einer Wirtszelle, welche mit einem Polynucleotid, dass eine Cyclodipeptidsynthetase, wie definiert in Anspruch 6, codiert, modifiziert ist, zur Herstellung von Cyclodipeptiden Cyclo(Tyr-Xaa), wobei Xaa ausgewählt wird aus der Gruppe, bestehend aus Tyr, Phe, Trp und Ala.

8. Verfahren zur Synthese von Cyclo(Tyr-Xaa)-cylodipeptiden, wobei Xaa aus der Gruppe ausgewählt ist, welche aus Tyr, Phe, Trp und Ala besteht, **dadurch gekennzeichnet, dass** es umfasst:
(1) Inkubieren zweier Aminosäuren Tyr und Xaa unter geeigneten Bedingungen mit einer Cyclodipeptidsynthetase, welche aus der Gruppe bestehend aus SEQ ID NO: 3, 4 und 6 ausgewählt wird, und
(2) Gewinnen der Cyclo(Tyr-Xaa)-cyclodipeptide, die hierdurch erhalten werden.

9. Verfahren zur Synthese von α,β-dehydrierten Cyclo(Tyr-Xaa)-cyclodipeptiden, wobei Xaa aus der Gruppe ausgewählt wird, welche aus Tyr, Phe, Trp und Ala besteht und **dadurch gekennzeichnet ist, dass** es umfasst:
(1') Inkubieren zweier Aminosäuren Tyr und Xaa unter geeigneten Bedingungen mit einer Cyclodipeptidsynthetase, welche aus der Gruppe ausgewählt wird, die aus SEQ ID NO: 3, 4 und 6 besteht, und einer gereinigten CDO und
(2') Gewinnen der α,β-dehydrierten Cyclodipeptide.

10. Verfahren gemäß Anspruch 8 oder Anspruch 9, wobei Schritt (1) oder Schritt (1') in Anwesenheit geeigneter Aminosäuren in einer Konzentration zwischen 0,1 mM bis 100 mM, bevorzugt von 1 mM bis 10 mM, einer Cyclodipeptidsynthetase in einer Konzentration zwischen 0,1 nM und 100 µM, bevorzugt von 1 µM bis 100 µM, in einem Puffer mit einem pH-Wert zwischen 6 und 8, welcher ein lösliches Extrakt prokaryontischer Zellen, zum Beispiel E. coli oder Streptomyces-Zellen enthält, welche keine Cyclodipeptidsynthetase produzieren, durchgeführt wird.

11. Verfahren gemäß einem der Ansprüche 8 bis 10, **dadurch gekennzeichnet, dass** es des Weiteren einen vorangehenden Schritt (P) umfasst, welcher in der Verwendung eines Polynucleotids, ausgewählt aus der Gruppe, bestehend aus SEQ ID NO: 1, 2 und 5, besteht, zur Synthese einer Cyclodipeptidsynthetase, wobei dieser Schritt vor Schritt (1) oder Schritt (1') durchgeführt wird.

12. Verfahren zur Synthese von Cyclo(Tyr-Xaa)-cyclodipeptide, wobei Xaa aus der Gruppe ausgewählt wird, welche aus Tyr, Phe, Trp und Ala besteht, und das die folgenden Schritte umfasst:
(1') Kultivieren einer Wirtszelle, modifiziert mit einem Polynucleotid, welches eine Cyclodipeptidsynthetase codiert, ausgewählt aus der Gruppe, bestehend aus SEQ ID NO: 1, 2 und 5, unter geeigneten Kulturbedingungen für diesen Wirt und
(2') Gewinnen der Cyclodipeptide aus dem Kulturmedium.

13. Verfahren zur Synthese von α,β-dehydrierten Cyclo(Tyr-Xaa)-cyclodipeptiden, wobei Xaa aus der Gruppe ausgewählt wird, welche aus Tyr, Phe, Trp und Ala besteht, umfassend die folgenden Schritte:
(1') Kultivieren einer Wirtszelle, modifiziert mit einem Polynucleotid, welches eine Cyclodipeptidsynthetase codiert, ausgewählt aus der Gruppe, bestehend aus SEQ ID NO: 1, 2 und 5, unter geeigneten Kulturbedingungen für diesen Wirt,
(1") Inkubieren des Cyclo(Tyr-Xaa)-cyclodipeptids, erhalten aus Schritt (1'), mit einer gereinigten CDO, und
(2') Gewinnen der Cyclodipeptide aus dem Kulturmedium.

## Revendications

1. Cyclodipeptide synthétase isolée, **caractérisée en ce que** :
- elle a la capacité à produire des cyclo(Tyr-Xaa) cyclodipeptides à partir de deux acides aminés Tyr et Xaa, Xaa étant un acide aminé quelconque, et
- elle consiste en un polypeptide choisi dans le groupe consistant en SEQ ID N° 3 et SEQ ID N° 6.

2. Polynucléotide isolé, **caractérisé en ce qu'**il est choisi parmi :
a) un polynucléotide codant pour une cyclodipeptide synthétase, choisi dans le groupe consistant en SEQ ID N° 1 et SEQ ID N° 5 ;
b) un polynucléotide complémentaire du polynucléotide a).

3. Vecteur recombinant comprenant un polynucléotide tel que défini dans la revendication 2, ou plasmide comprenant ledit polynucléotide.

4. Cellule hôte modifiée par un polynucléotide tel que défini dans la revendication 2 ou par un vecteur recombinant tel que défini dans la revendication 3.

5. Cellule hôte selon la revendication 4, choisie dans le groupe consistant en une cellule procaryote, de préférence une bactérie.

6. Utilisation d'une cyclodipeptide synthétase, **caractérisée en ce qu'**elle a la capacité de produire des cyclo(Tyr-Xaa) cyclodipeptides à partir de deux acides aminés Tyr et Xaa, et qu'elle consiste en une séquence polypeptidique choisie dans le groupe consistant en SEQ ID N° 3, 4 et 6, pour produire des cyclodipeptides cyclo(Tyr-Xaa), Xaa étant choisi dans le groupe consistant en Tyr, Phe, Trp et Ala.

7. Utilisation d'une cellule hôte modifiée par un polynucléotide codant pour une cyclodipeptide synthétase telle que définie dans la revendication 6, pour produire des cyclodipeptides cyclo(Tyr-Xaa), Xaa étant choisi dans le groupe consistant en Tyr, Phe, Trp et Ala.

8. Procédé de synthèse de cyclo(Tyr-Xaa) cyclodipeptides, où Xaa est choisi dans le groupe consistant en Tyr, Phe, Trp et Ala, **caractérisé en ce qu'**il comprend :
(1) l'incubation de deux acides aminés Tyr et Xaa dans des conditions appropriées, avec une cyclodipeptide synthétase choisie dans le groupe consistant en SEQ ID N° 3, 4 et 6, et
(2) la récupération des cyclo(Tyr-Xaa) cyclodipeptides ainsi obtenus.

9. Procédé de synthèse de cyclo(Tyr-Xaa) cyclodipeptides α,β-déshydrogénés, où Xaa est choisi dans le groupe consistant en Tyr, Phe, Trp et Ala, **caractérisé en ce qu'**il comprend :
(1') l'incubation de deux acides aminés Tyr et Xaa dans des conditions appropriées avec une cyclodipeptide synthétase choisie dans le groupe consistant en SEQ ID N° 3, 4 et 6, et une CDO purifiée, et
(2') la récupération des cyclodipeptides α,β-déshydrogénés.

10. Procédé selon la revendication 8 ou 9, dans lequel l'étape (1) ou l'étape (1') est mise en oeuvre en présence d'acides aminés appropriés à une concentration comprise entre 0,1 mM et 100 mM, de préférence entre 1 mM et 10 mM, une cyclodipeptide synthétase à une concentration comprise entre 0,1 nM et 100 µM, de préférence entre 1 µM et 100 µM, dans un tampon à un pH compris entre 6 et 8, et contenant un extrait soluble de cellules procaryotes telles que des cellules de *E*. *coli* ou de *Streptomyces* qui ne produisent pas de cyclodipeptide synthétase.

11. Procédé selon l'une quelconque des revendications 8 à 10, **caractérisé en ce qu'**il comprend en outre une étape préliminaire (P) consistant en l'utilisation d'un polynucléotide choisi dans le groupe consistant en SEQ ID N° 1, 2 et 5, pour synthétiser une cyclodipeptide synthétase, qui est mise en oeuvre avant l'étape (1) ou l'étape (1').

12. Procédé de synthèse de cyclo(Tyr-Xaa) cyclodipeptides, où Xaa est choisi dans le groupe consistant en Tyr, Phe, Trp et Ala, comprenant les étapes suivantes :
(1') culture d'une cellule hôte modifiée par un polynucléotide codant pour une cyclodipeptide synthétase, choisi dans le groupe consistant en SEQ ID N° 1, 2 et 5 dans des conditions de culture appropriées audit hôte, et
(2') la récupération des cyclodipeptides à partir du milieu de culture.

13. Procédé de synthèse de cyclo(Tyr-Xaa) cyclodipeptides α,β-déshydrogénés, où Xaa est choisi dans le groupe consistant en Tyr, Phe, Trp et Ala, comprenant les étapes suivantes :
(1') culture d'une cellule hôte modifiée par un polynucléotide codant pour une cyclodipeptide synthétase, choisi dans le groupe consistant en SEQ ID N° 1, 2 et 5 dans des conditions de culture appropriées audit hôte,
(1") l'incubation du cyclo(Tyr-Xaa) cyclodipeptide obtenu à l'étape (1') avec une CDO purifiée, et
(2") la récupération des cyclodipeptides à partir du milieu de culture.


### SEQUENCE LISTING

<110> Commissariat à l'énergie atomique
   Kyowa Hakko Kogyo Co., Ltd.
   Universite Paris sud 11
   Belin, Pascal
   Gondry, Muriel
   Thai, Robert
   Pernodet, Jean-Luc
   Genet, Roger
<120> Cyclodipeptide synthetase and its use for synthesis of cycle (Tyr-Xaa) cyclodipeptides
<130> F263PCT155
<160> 21
<170> PatentIn version 3.3
<210> 1
   <211> 726
   <212> DNA
   <213> Mycobacterium tuberculosis
<400> 1
   atgsagctag gcaggcgcat tccggaagcc accgcccagg aagggtttct ggttcggcca     60
   ttcacccaac aatgtcagat catccacacc gaaggagatc atgctgttat cggggtatcc    120
   ccggggaaca gttacttctc ccgccagcgc ctacgggatc tcgggctttg gggtctcacg    180
   aattttgatc gtgtggactt cgtctacacc gatgtccatg tcgccgagag ttacgaagcg    240
   ctaggcgatt ccgcaatcga agcccggcgc aaggcggtca aaaacatccg cggcgtccgc    300
   gccaagatca ccaccacggt gaacgaactc gatccggccg gggcccggct gtgcgttcgt    360
   ccgatgtcgg agttccagtc caacgaggca taccgggagc tgcatgcgga cctgctcacg    420
   cgcctgaaag acgacgagga cttgcgcgcc gtctgccagg acctagtgcg gcgcttcctg    480
   tccacgaaag tgggtccgcg gcagggggcg acggctactc aagagcaggt gtgcatggac    540
   tacatttgcg ccgaggcccc gctattcctc gacacacctg cgattctcgg agtgccgtcg    600
   tcgttgaatt gctaccacca atcactgccc ctcgccgaaa tgctctacgc ccgaggatcg    660
   ggactacggg catcgcgcaa tcaaggccac gccattgtta cccctgatgg gagccccgcc    720
   gaatga                                                               726
<210> 2
   <211> 870
   <212> DNA
   <213> Mycobacterium tuberculosis
<400> 2
   atgtcatacg tggctgccga accaggcgtg ctgatctcgc cgacggacga cttgcagagc    60
   ccccggtcag ccccggcagc gcatgacgaa aatgcggacg gcataacagg cgggaccaga   120
   gacgactctg ctcccaactc acggtttcag ctaggcaggc gcattccgga agccaccgcc   180
   caggaagggt ttctggttcg gccattcacc caacaatgtc agatcatcca caccgaagga   240
   gatcatgctg ttatcggggt atccccgggg aacagttact tctcccgcca gcgcctacgg   300
   gatctcgggc tttggggtct cacgaatttt gatcgtgtgg acttcgtcta caccgatgtc   360
   catgtcgccg agagttacga agcgctaggc gattccgcaa tcgaagcccg gcgcaaggcg   420
   gtcaaaaaca tccgcggcgt ccgcgccaag atcaccacca cggtgaacga actcgatccg   480
   gccggggccc ggctgtgcgt tcgtccgatg tcggagttcc agtccaacga ggcataccgg   540
   gagctgcatg cggacctgct cacgcgcctg aaagacgacg aggacttgcg cgccgtctgc   600
   caggacctag tgcggcgctt cctgtccacg aaagtgggtc cgcggcaggg ggcgacggct   660
   actcaagagc aggtgtgcat ggactacatt tgcgccgagg ccccgctatt cctcgacaca   720
   cctgcgattc tcggagtgcc gtcgtcgttg aattgctacc accaatcact gcccctcgcc   780
   gaaatgctct acgcccgagg atcgggacta cgggcatcgc gcaatcaagg ccacgccatt   840
   gttacccctg atgggagccc cgccgaatga                                     870
<210> 3
   <211> 241
   <212> PRT
   <213> Mycobacterium tuberculosis
<220>
   <221> MISC_FEATURE
   <222> (2)..(2)
   <223> Xaa is Gln or Glu
<400> 3
   Met Xaa Leu Gly Arg Arg Ile Pro Glu Ala Thr Ala Gln Glu Gly Phe
   1               5                   10                  15
   Leu Val Arg Pro Phe Thr Gln Gln Cys Gln Ile Ile His Thr Glu Gly
               20                  25                  30
   Asp His Ala Val Ile Gly Val Ser Pro Gly Asn Ser Tyr Phe Ser Arg
           35                  40                  45
   Gln Arg Leu Arg Asp Leu Gly Leu Trp Gly Leu Thr Asn Phe Asp Arg
       50                  55                  60
   Val Asp Phe Val Tyr Thr Asp Val His Val Ala Glu Ser Tyr Glu Ala
   65                  70                  75                  80
   Leu Gly Asp Ser Ala Ile Glu Ala Arg Arg Lys Ala Val Lys Asn Ile
                   85                  90                  95
   Arg Gly Val Arg Ala Lys Ile Thr Thr Thr Val Asn Glu Leu Asp Pro
               100                 105                 110
   Ala Gly Ala Arg Leu Cys Val Arg Pro Met Ser Glu Phe Gln Ser Asn
           115                 120                 125
   Glu Ala Tyr Arg Glu Leu His Ala Asp Leu Leu Thr Arg Leu Lys Asp
       130                 135                 140
   Asp Glu Asp Leu Arg Ala Val Cys Gln Asp Leu Val Arg Arg Phe Leu
   145                 150                 155                 160
   Ser Thr Lys Val Gly Pro Arg Gln Gly Ala Thr Ala Thr Gln Glu Gln
                   165                 170                 175
   Val Cys Met Asp Tyr Ile Cys Ala Glu Ala Pro Leu Phe Leu Asp Thr
               180                 185                 190
   Pro Ala Ile Leu Gly Val Pro Ser Ser Leu Asn Cys Tyr His Gln Ser
           195                 200                 205
   Leu Pro Leu Ala Glu Met Leu Tyr Ala Arg Gly Ser Gly Leu Arg Ala
       210                 215                 220
   Ser Arg Asn Gln Gly His Ala Ile Val Thr Pro Asp Gly Ser Pro Ala
   225                 230                 235                 240
   Glu
<210> 4
   <211> 289
   <212> PRT
   <213> Mycobacterium tuberculosis
<400> 4
   Met Ser Tyr Val Ala Ala Glu Pro Gly Val Leu Ile Ser Pro Thr Asp
   1               5                   10                  15
   Asp Leu Gln Ser Pro Arg Ser Ala Pro Ala Ala His Asp Glu Asn Ala
               20                  25                  30
   Asp Gly Ile Thr Gly Gly Thr Arg Asp Asp Ser Ala Pro Asn Ser Arg
           35                  40                  45
   Phe Gln Leu Gly Arg Arg Ile Pro Glu Ala Thr Ala Gln Glu Gly Phe
       50                  55                  60
   Leu Val Arg Pro Phe Thr Gln Gln Cys Gln Ile Ile His Thr Glu Gly
   65                  70                  75                  80
   Asp His Ala Val Ile Gly Val Ser Pro Gly Asn Ser Tyr Phe Ser Arg
                   85                  90                  95
   Gln Arg Leu Arg Asp Leu Gly Leu Trp Gly Leu Thr Asn Phe Asp Arg
               100                 105                 110
   Val Asp Phe Val Tyr Thr Asp Val His Val Ala Glu Ser Tyr Glu Ala
           115                 120                 125
   Leu Gly Asp Ser Ala Ile Glu Ala Arg Arg Lys Ala Val Lys Asn Ile
   130 135 140
   Arg Gly Val Arg Ala Lys Ile Thr Thr Thr Val Asn Glu Leu Asp Pro
   145 150 155 160
   Ala Gly Ala Arg Leu Cys Val Arg Pro Met Ser Glu Phe Gln Ser Asn
   165 170 175
   Glu Ala Tyr Arg Glu Leu His Ala Asp Leu Leu Thr Arg Leu Lys Asp
   180 185 190
   Asp Glu Asp Leu Arg Ala Val Cys Gln Asp Leu Val Arg Arg Phe Leu
   195 200 205
   Ser Thr Lys Val Gly Pro Arg Gln Gly Ala Thr Ala Thr Gln Glu Gln
   210 215 220
   Val Cys Met Asp Tyr Ile Cys Ala Glu Ala Pro Leu Phe Leu Asp Thr
   225 230 235 240
   Pro Ala Ile Leu Gly Val Pro Ser Ser Leu Asn Cys Tyr His Gln Ser
   245 250 255
   Leu Pro Leu Ala Glu Met Leu Tyr Ala Arg Gly Ser Gly Leu Arg Ala
   260 265 270
   Ser Arg Asn Gln Gly His Ala Ile Val Thr Pro Asp Gly Ser Pro Ala
   275 280 285
   Glu
<210> 5
   <211> 870
   <212> DNA
   <213> Mycobacterium tuberculosis
<220>
   <221> CDS
   <222> (1)..(870)
<400> 5
   atg gca tac gtg gct gcc gaa cca ggc gtg ctg atc tcg ccg acg gac      48
   Met Ala Tyr Val Ala Ala Glu Pro Gly Val Leu Ile Ser Pro Thr Asp
   1               5                   10                  15
   gac ttg cag agc ccc cgg tca gcc ccg gca gcg cat gac gaa aat gcg      96
   Asp Leu Gln Ser Pro Arg Ser Ala Pro Ala Ala His Asp Glu Asn Ala
               20                  25                  30
   gac ggc ata aca ggc ggg acc aga gac gac tct gct ccc aac tca cgg     144
   Asp Gly Ile Thr Gly Gly Thr Arg Asp Asp Ser Ala Pro Asn Ser Arg
           35                  40                  45
   ttt cag cta ggc agg cgc att ccg gaa gcc acc gcc cag gaa ggg ttt      192
   Phe Gln Leu Gly Arg Arg Ile Pro Glu Ala Thr Ala Gln Glu Gly Phe
       50                  55                  60
   ctg gtt cgg cca ttc acc caa caa tgt cag atc atc cac acc gaa gga      240
   Leu Val Arg Pro Phe Thr Gln Gln Cys Gln Ile Ile His Thr Glu Gly
   65                  70                  75                  80
   gat cat gct gtt atc ggg gta tcc ccg ggg aac agt tac ttc tcc cgc      288
   Asp His Ala Val Ile Gly Val Ser Pro Gly Asn Ser Tyr Phe Ser Arg
                   85                  90                  95
   cag cgc cta cgg gat ctc ggg ctt tgg ggt ctc acg aat ttt gat cgt      336
   Gln Arg Leu Arg Asp Leu Gly Leu Trp Gly Leu Thr Asn Phe Asp Arg
               100                 105                 110
   gtg gac ttc gtc tac acc gat gtc cat gtc gcc gag agt tac gaa gcg      384
   Val Asp Phe Val Tyr Thr Asp Val His Val Ala Glu Ser Tyr Glu Ala
           115                 120                 125
   cta ggc gat tcc gca atc gaa gcc cgg cgc aag gcg gtc aaa aac atc      432
   Leu Gly Asp Ser Ala Ile Glu Ala Arg Arg Lys Ala Val Lys Asn Ile
       130                 135                 140
   cgc ggc gtc cgc gcc aag atc acc acc acg gtg aac gaa ctc gat ccg      480
   Arg Gly Val Arg Ala Lys Ile Thr Thr Thr Val Asn Glu Leu Asp Pro
   145                 150                 155                 160
   gcc ggg gcc cgg ctg tgc gtt cgt ccg atg tcg gag ttc cag tcc aac      528
   Ala Gly Ala Arg Leu Cys Val Arg Pro Met Ser Glu Phe Gln Ser Asn
                   165                 170                 175
   gag gca tac cgg gag ctg cat gcg gac ctg ctc acg cgc ctg aaa gac      576
   Glu Ala Tyr Arg Glu Leu His Ala Asp Leu Leu Thr Arg Leu Lys Asp
               180                 185                 190
   gac gag gac ttg cgc gcc gtc tgc cag gac cta gtg cgg cgc ttc ctg      624
   Asp Glu Asp Leu Arg Ala Val Cys Gln Asp Leu Val Arg Arg Phe Leu
           195                 200                 205
   tcc acg aaa gtg ggt ccg cgg cag ggg gcg acg gct act caa gag cag      672
   Ser Thr Lys Val Gly Pro Arg Gln Gly Ala Thr Ala Thr Gln Glu Gln
       210                 215                 220
   gtg tgc atg gac tac att tgc gcc gag gcc ccg cta ttc ctc gac aca      720
   Val Cys Met Asp Tyr Ile Cys Ala Glu Ala Pro Leu Phe Leu Asp Thr
   225                 230                 235                 240
   cct gcg att ctc gga gtg ccg tcg tcg ttg aat tgc tac cac caa tca      768
   Pro Ala Ile Leu Gly Val Pro Ser Ser Leu Asn Cys Tyr His Gln Ser
                   245                 250                 255
   ctg ccc ctc gcc gaa atg ctc tac gcc cga gga tcg gga cta cgg gca      816
   Leu Pro Leu Ala Glu Met Leu Tyr Ala Arg Gly Ser Gly Leu Arg Ala
               260                 265                 270
   tcg cgc aat caa ggc cac gcc att gtt acc cct gat ggg agc ccc gcc      864
   Ser Arg Asn Gln Gly His Ala Ile Val Thr Pro Asp Gly Ser Pro Ala
           275                 280                 285
   gaa tga                                                              870
   Glu
<210> 6
   <211> 289
   <212> PRT
   <213> Mycobacterium tuberculosis
<220>
   <221> misc_feature
   <222> (2)..(2)
   <223> The 'Xaa' at location 2 stands for Ala.
<400> 6
   Met Ala Tyr Val Ala Ala Glu Pro Gly Val Leu Ile Ser Pro Thr Asp
   1               5                   10                  15
   Asp Leu Gln Ser Pro Arg Ser Ala Pro Ala Ala His Asp Glu Asn Ala
               20                  25                  30
   Asp Gly Ile Thr Gly Gly Thr Arg Asp Asp Ser Ala Pro Asn Ser Arg
           35                  40                  45
   Phe Gln Leu Gly Arg Arg Ile Pro Glu Ala Thr Ala Gln Glu Gly Phe
       50                  55                  60
   Leu Val Arg Pro Phe Thr Gln Gln Cys Gln Ile Ile His Thr Glu Gly
   65                  70                  75                  80
   Asp His Ala Val Ile Gly Val Ser Pro Gly Asn Ser Tyr Phe Ser Arg
                   85                  90                  95
   Gln Arg Leu Arg Asp Leu Gly Leu Trp Gly Leu Thr Asn Phe Asp Arg
               100                 105                 110
   Val Asp Phe Val Tyr Thr Asp Val His Val Ala Glu Ser Tyr Glu Ala
           115                 120                 125
   Leu Gly Asp Ser Ala Ile Glu Ala Arg Arg Lys Ala Val Lys Asn Ile
       130                 135                 140
   Arg Gly Val Arg Ala Lys Ile Thr Thr Thr Val Asn Glu Leu Asp Pro
   145                 150                 155                 160
   Ala Gly Ala Arg Leu Cys Val Arg Pro Met Ser Glu Phe Gln Ser Asn
                   165                 170                 175
   Glu Ala Tyr Arg Glu Leu His Ala Asp Leu Leu Thr Arg Leu Lys Asp
               180                 185                 190
   Asp Glu Asp Leu Arg Ala Val Cys Gln Asp Leu Val Arg Arg Phe Leu
           195                 200                 205
   Ser Thr Lys Val Gly Pro Arg Gln Gly Ala Thr Ala Thr Gln Glu Gln
       210                 215                 220
   Val Cys Met Asp Tyr Ile Cys Ala Glu Ala Pro Leu Phe Leu Asp Thr
   225                 230                 235                 240
   Pro Ala Ile Leu Gly Val Pro Ser Ser Leu Asn Cys Tyr His Gln Ser
                   245                 250                 255
   Leu Pro Leu Ala Glu Met Leu Tyr Ala Arg Gly Ser Gly Leu Arg Ala
               260                 265                 270
   Ser Arg Asn Gln Gly His Ala Ile Val Thr Pro Asp Gly Ser Pro Ala
           275                 280                 285
   Glu
<210> 7
   <211> 35
   <212> DNA
   <213> Artificial
<220>
   <223> Primer A
<400> 7
   ccgtccctat ggtccaagga aaacaatgtc atacg 35
<210> 8
   <211> 34
   <212> DNA
   <213> Artificial
<220>
   <223> Primer _{B}
<400> 8
   gcaagcaata acggcggggc tcccatcagg ggta 34
<210> 9
   <211> 42
   <212> DNA
   <213> Artificial
<220>
   <223> Primer c
<400> 9
   ggcttcgaga atctttattt tcagggctca tacgtggctg cc 42
<210> 10
   <211> 47
   <212> DNA
   <213> Artificial
<220>
   <223> Primer D
<400> 10
   ggggaccact ttgtacaaga aagctgggtc cttattcggc ggggctc 47
<210> 11
   <211> 47
   <212> DNA
   <213> Artificial
<220>
   <223> Primer E
<400> 11
   ggggacaagt ttgtacaaaa aagcaggctt cgagaatctt tattttc 47
<210> 12
   <211> 20
   <212> DNA
   <213> Artificial
<220>
   <223> Primer F
<400> 12
   tcggccattc acccaacaat 20
<210> 13
   <211> 15
   <212> DNA
   <213> Artificial
<220>
   <223> Froward primer M13
<400> 13
   gtaaacgacg gccag 15
<210> 14
   <211> 17
   <212> DNA
   <213> Artificial
<220>
   <223> Reverse primer M13
<400> 14
   caggaaacag ctatgac 17
<210> 15
   <211> 32
   <212> DNA
   <213> Artificial
<220>
   <223> Primer KRVLF
<400> 15
   cggccatggc atacgtggct gccgaaccag gc 32
<210> 16
   <211> 30
   <212> DNA
   <213> Artificial
<220>
   <223> Primer KRVR
<400> 16
   ggcagatctt tcggcggggc tcccatcagg 30
<210> 17
   <211> 34
   <212> DNA
   <213> Artificial
<220>
   <223> Primer KRVCF
<400> 17
   cggccatgga gctaggcagg cgcattccgg aagc 34
<210> 18
   <211> 29
   <212> PRT
   <213> Artificial
<220>
   <223> N-terminal extension of Rv2275
<400> 18
   Met Ser Tyr Tyr His His His His His His Leu Glu Ser Thr Ser Leu
   1               5                   10                  15
   Tyr Lys Lys Ala Gly Phe Glu Asn Leu Tyr Phe Gln Gly
               20                  25
<210> 19
   <211> 5591
   <212> DNA
   <213> Artificial
<220>
   <223> DNA sequence of expression vector pEXP-Rv2275
<400> 19
   agatctcgat cccgcgaaat taatacgact cactataggg agaccacaac ggtttccctc    60
   tagaaataat tttgtttaac tttaagaagg agatatacat atgtcgtact accatcacca   120
   tcaccatcac ctcgaatcaa caagtttgta caaaaaagca ggcttcgaga atctttattt   180
   tcagggctca tacgtggctg ccgaaccagg cgtgctgatc tcgccgacgg acgacttgca   240
   gagcccccgg tcagccccgg cagcgcatga cgaaaatgcg gacggcataa caggcgggac   300
   cagagacgac tctgctccca actcacggtt tcagctaggc aggcgcattc cggaagccac   360
   cgcccaggaa gggtttctgg ttcggccatt cacccaacaa tgtcagatca tccacaccga   420
   aggagatcat gctgttatcg gggtatcccc ggggaacagt tacttctccc gccagcgcct   480
   acgggatctc gggctttggg gtctcacgaa ttttgatcgt gtggacttcg tctacaccga   540
   tgtccatgtc gccgagagtt acgaagcgct aggcgattcc gcaatcgaag cccggcgcaa   600
   ggcggtcaaa aacatccgcg gcgtccgcgc caagatcacc accacggtga acgaactcga   660
   tccggccggg gcccggctgt gcgttcgtcc gatgtcggag ttccagtcca acgaggcata   720
   ccgggagctg catgcggacc tgctcacgcg cctgaaagac gacgaggact tgcgcgccgt   780
   ctgccaggac ctagtgcggc gcttcctgtc cacgaaagtg ggtccgcggc agggggcgac   840
   ggctactcaa gagcaggtgt gcatggacta catttgcgcc gaggccccgc tattcctcga 900
   cacacctgcg attctcggag tgccgtcgtc gttgaattgc taccaccaat cactgcccct 960
   cgccgaaatg ctctacgccc gaggatcggg actacgggca tcgcgcaatc aaggccacgc 1020
   cattgttacc cctgatggga gccccgccga ataaggaccc agctttcttg tacaaagtgg 1080
   ttgattcgag gctgctaaca aagcccgaaa ggaagctgag ttggctgctg ccaccgctga 1140
   gcaataacta gcataacccc ttggggcctc taaacgggtc ttgaggggtt ttttgctgaa 1200
   aggaggaact atatccggat atccacagga cgggtgtggt cgccatgatc gcgtagtcga 1260
   tagtggctcc aagtagcgaa gcgagcagga ctgggcggcg gccaaagcgg tcggacagtg 1320
   ctccgagaac gggtgcgcat agaaattgca tcaacgcata tagcgctagc agcacgccat 1380
   agtgactggc gatgctgtcg gaatggacga tatcccgcaa gaggcccggc agtaccggca 1440
   taaccaagcc tatgcctaca gcatccaggg tgacggtgcc gaggatgacg atgagcgcat 1500
   tgttagattt catacacggt gcctgactgc gttagcaatt taactgtgat aaactaccgc 1560
   attaaagctt atcgatgata agctgtcaaa catgagaatt cttgaagacg aaagggcctc 1620
   gtgatacgcc tatttttata ggttaatgtc atgataataa tggtttctta gacgtcaggt 1680
   ggcacttttc ggggaaatgt gcgcggaacc cctatttgtt tatttttcta aatacattca 1740
   aatatgtatc cgctcatgag acaataaccc tgataaatgc ttcaataata ttgaaaaagg 1800
   aagagtatga gtattcaaca tttccgtgtc gcccttattc ccttttttgc ggcattttgc 1860
   cttcctgttt ttgctcaccc agaaacgctg gtgaaagtaa aagatgctga agatcagttg 1920
   ggtgcacgag tgggttacat cgaactggat ctcaacagcg gtaagatcct tgagagtttt 1980
   cgccccgaag aacgttttcc aatgatgagc acttttaaag ttctgctatg tggcgcggta 2040
   ttatcccgtg ttgacgccgg gcaagagcaa ctcggtcgcc gcatacacta ttctcagaat 2100
   gacttggttg agtactcacc agtcacagaa aagcatctta cggatggcat gacagtaaga 2160
   gaattatgca gtgctgccat aaccatgagt gataacactg cggccaactt acttctgaca 2220
   acgatcggag gaccgaagga gctaaccgct tttttgcaca acatggggga tcatgtaact 2280
   cgccttgatc gttgggaacc ggagctgaat gaagccatac caaacgacga gcgtgacacc 2340
   acgatgcctg cagcaatggc aacaacgttg cgcaaactat taactggcga actacttact 2400
   ctagcttccc ggcaacaatt aatagactgg atggaggcgg ataaagttgc aggaccactt 2460
   ctgcgctcgg cccttccggc tggctggttt attgctgata aatctggagc cggtgagcgt 2520
   gggtctcgcg gtatcattgc agcactgggg ccagatggta agccctcccg tatcgtagtt 2580
   atctacacga cggggagtca ggcaactatg gatgaacgaa atagacagat cgctgagata 2640
   ggtgcctcac tgattaagca ttggtaactg tcagaccaag tttactcata tatactttag 2700
   attgatttaa aacttcattt ttaatttaaa aggatctagg tgaagatcct ttttgataat 2760
   ctcatgacca aaatccctta acgtgagttt tcgttccact gagcgtcaga ccccgtagaa 2820
   aagatcaaag gatcttcttg agatcctttt tttctgcgcg taatctgctg cttgcaaaca 2880
   aaaaaaccac cgctaccagc ggtggtttgt ttgccggatc aagagctacc aactcttttt 2940
   ccgaaggtaa ctggcttcag cagagcgcag ataccaaata ctgtccttct agtgtagccg 3000
   tagttaggcc accacttcaa gaactctgta gcaccgccta catacctcgc tctgctaatc 3060
   ctgttaccag tggctgctgc cagtggcgat aagtcgtgtc ttaccgggtt ggactcaaga 3120
   cgatagttac cggataaggc gcagcggtcg ggctgaacgg ggggttcgtg cacacagccc 3180
   agcttggagc gaacgaccta caccgaactg agatacctac agcgtgagct atgagaaagc 3240
   gccacgcttc ccgaagggag aaaggcggac aggtatccgg taagcggcag ggtcggaaca 3300
   ggagagcgca cgagggagct tccaggggga aacgcctggt atctttatag tcctgtcggg 3360
   tttcgccacc tctgacttga gcgtcgattt ttgtgatgct cgtcaggggg gcggagccta 3420
   tggaaaaacg ccagcaacgc ggccttttta cggttcctgg ccttttgctg gccttttgct 3480
   cacatgttct ttcctgcgtt atcccctgat tctgtggata accgtattac cgcctttgag 3540
   tgagctgata ccgctcgccg cagccgaacg accgagcgca gcgagtcagt gagcgaggaa 3600
   gcggaagagc gcctgatgcg gtattttctc cttacgcatc tgtgcggtat ttcacaccgc 3660
   atatatggtg cactctcagt acaatctgct ctgatgccgc atagttaagc cagtatacac 3720
   tccgctatcg ctacgtgact gggtcatggc tgcgccccga cacccgccaa cacccgctga 3780
   cgcgccctga cgggcttgtc tgctcccggc atccgcttac agacaagctg tgaccgtctc 3840
   cgggagctgc atgtgtcaga ggttttcacc gtcatcaccg aaacgcgcga ggcagctgcg 3900
   gtaaagctca tcagcgtggt cgtgaagcga ttcacagatg tctgcctgtt catccgcgtc 3960
   cagctcgttg agtttctcca gaagcgttaa tgtctggctt ctgataaagc gggccatgtt 4020
   aagggcggtt ttttcctgtt tggtcactga tgcctccgtg taagggggat ttctgttcat 4080
   gggggtaatg ataccgatga aacgagagag gatgctcacg atacgggtta ctgatgatga 4140
   acatgcccgg ttactggaac gttgtgaggg taaacaactg gcggtatgga tgcggcggga 4200
   ccagagaaaa atcactcagg gtcaatgcca gcgcttcgtt aatacagatg taggtgttcc 4260
   acagggtagc cagcagcatc ctgcgatgca gatccggaac ataatggtgc agggcgctga 4320
   cttccgcgtt tccagacttt acgaaacacg gaaaccgaag accattcatg ttgttgctca 4380
   ggtcgcagac gttttgcagc agcagtcgct tcacgttcgc tcgcgtatcg gtgattcatt 4440
   ctgctaacca gtaaggcaac cccgccagcc tagccgggtc ctcaacgaca ggagcacgat 4500
   catgcgcacc cgtggccagg acccaacgct gcccgagatg cgccgcgtgc ggctgctgga 4560
   gatggcggac gcgatggata tgttctgcca agggttggtt tgcgcattca cagttctccg 4620
   caagaattga ttggctccaa ttcttggagt ggtgaatccg ttagcgaggt gccgccggct 4680
   tccattcagg tcgaggtggc ccggctccat gcaccgcgac gcaacgcggg gaggcagaca 4740
   aggtataggg cggcgcctac aatccatgcc aacccgttcc atgtgctcgc cgaggcggca 4800
   taaatcgccg tgacgatcag cggtccagtg atcgaagtta ggctggtaag agccgcgagc 4860
   gatccttgaa gctgtccctg atggtcgtca tctacctgcc tggacagcat ggcctgcaac 4920
   gcgggcatcc cgatgccgcc ggaagcgaga agaatcataa tggggaaggc catccagcct   4980
   cgcgtcgcga acgccagcaa gacgtagccc agcgcgtcgg ccgccatgcc ggcgataatg   5040
   gcctgcttct cgccgaaacg tttggtggcg ggaccagtga cgaaggcttg agcgagggcg   5100
   tgcaagattc cgaataccgc aagcgacagg ccgatcatcg tcgcgctcca gcgaaagcgg   5160
   tcctcgccga aaatgaccca gagcgctgcc ggcacctgtc ctacgagttg catgataaag   5220
   aagacagtca taagtgcggc gacgatagtc atgccccgcg cccaccggaa ggagctgact   5280
   gggttgaagg ctctcaaggg catcggtcga tcgacgctct cccttatgcg actcctgcat   5340
   taggaagcag cccagtagta ggttgaggcc gttgagcacc gccgccgcaa ggaatggtgc   5400
   atgcaaggag atggcgccca acagtccccc ggccacgggg cctgccacca tacccacgcc   5460
   gaaacaagcg ctcatgagcc cgaagtggcg agcccgatct tccccatcgg tgatgtcggc   5520
   gatataggcg ccagcaaccg cacctgtggc gccggtgatg ccggccacga tgcgtccggc   5580
   gtagaggatc g                                                          5591
<210> 20
   <211> 4286
   <212> DNA
   <213> Artificial
<220>
   <223> DNA sequence of plasmid pQE60-Rv2275L
<400> 20
   ctcgagaaat cataaaaaat ttatttgctt tgtgagcgga taacaattat aatagattca    60
   attgtgagcg gataacaatt tcacacagaa ttcattaaag aggagaaatt aaccatggca   120
   tacgtggctg ccgaaccagg cgtgctgatc tcgccgacgg acgacttgca gagcccccgg   180
   tcagccccgg cagcgcatga cgaaaatgcg gacggcataa caggcgggac cagagacgac   240
   tctgctccca actcacggtt tcagctaggc aggcgcattc cggaagccac cgcccaggaa   300
   gggtttctgg ttcggccatt cacccaacaa tgtcagatca tccacaccga aggagatcat   360
   gctgttatcg gggtatcccc ggggaacagt tacttctccc gccagcgcct acgggatctc   420
   gggctttggg gtctcacgaa ttttgatcgt gtggacttcg tctacaccga tgtccatgtc   480
   gccgagagtt acgaagcgct aggcgattcc gcaatcgaag cccggcgcaa ggcggtcaaa   540
   aacatccgcg gcgtccgcgc caagatcacc accacggtga acgaactcga tccggccggg   600
   gcccggctgt gcgttcgtcc gatgtcggag ttccagtcca acgaggcata ccgggagctg   660
   catgcggacc tgctcacgcg cctgaaagac gacgaggact tgcgcgccgt ctgccaggac   720
   ctagtgcggc gcttcctgtc cacgaaagtg ggtccgcggc agggggcgac ggctactcaa   780
   gagcaggtgt gcatggacta catttgcgcc gaggccccgc tattcctcga cacacctgcg   840
   attctcggag tgccgtcgtc gttgaattgc taccaccaat cactgcccct cgccgaaatg   900
   ctctacgccc gaggatcggg actacgggca tcgcgcaatc aaggccacgc cattgttacc   960
   cctgatggga gccccgccga aagatctcat caccatcacc atcactaagc ttaattagct  1020
   gagcttggac tcctgttgat agatccagta atgacctcag aactccatct ggatttgttc 1080
   agaacgctcg gttgccgccg ggcgtttttt attggtgaga atccaagcta gcttggcgag 1140
   attttcagga gctaaggaag ctaaaatgga gaaaaaaatc actggatata ccaccgttga 1200
   tatatcccaa tggcatcgta aagaacattt tgaggcattt cagtcagttg ctcaatgtac 1260
   ctataaccag accgttcagc tggatattac ggccttttta aagaccgtaa agaaaaataa 1320
   gcacaagttt tatccggcct ttattcacat tcttgcccgc ctgatgaatg ctcatccgga 1380
   atttcgtatg gcaatgaaag acggtgagct ggtgatatgg gatagtgttc acccttgtta 1440
   caccgttttc catgagcaaa ctgaaacgtt ttcatcgctc tggagtgaat accacgacga 1500
   tttccggcag tttctacaca tatattcgca agatgtggcg tgttacggtg aaaacctggc 1560
   ctatttccct aaagggttta ttgagaatat gtttttcgtc tcagccaatc cctgggtgag 1620
   tttcaccagt tttgatttaa acgtggccaa tatggacaac ttcttcgccc ccgttttcac 1680
   catgcatggg caaatattat acgcaaggcg acaaggtgct gatgccgctg gcgattcagg 1740
   ttcatcatgc cgtctgtgat ggcttccatg tcggcagaat gcttaatgaa ttacaacagt 1800
   actgcgatga gtggcagggc ggggcgtaat ttttttaagg cagttattgg tgcccttaaa 1860
   cgcctggggt aatgactctc tagcttgagg catcaaataa aacgaaaggc tcagtcgaaa 1920
   gactgggcct ttcgttttat ctgttgtttg tcggtgaacg ctctcctgag taggacaaat 1980
   ccgccgctct agagctgcct cgcgcgtttc ggtgatgacg gtgaaaacct ctgacacatg 2040
   cagctcccgg agacggtcac agcttgtctg taagcggatg ccgggagcag acaagcccgt 2100
   cagggcgcgt cagcgggtgt tggcgggtgt cggggcgcag ccatgaccca gtcacgtagc 2160
   gatagcggag tgtatactgg cttaactatg cggcatcaga gcagattgta ctgagagtgc 2220
   accatatgcg gtgtgaaata ccgcacagat gcgtaaggag aaaataccgc atcaggcgct 2280
   cttccgcttc ctcgctcact gactcgctgc gctcggtcgt tcggctgcgg cgagcggtat 2340
   cagctcactc aaaggcggta atacggttat ccacagaatc aggggataac gcaggaaaga 2400
   acatgtgagc aaaaggccag caaaaggcca ggaaccgtaa aaaggccgcg ttgctggcgt 2460
   ttttccatag gctccgcccc cctgacgagc atcacaaaaa tcgacgctca agtcagaggt 2520
   ggcgaaaccc gacaggacta taaagatacc aggcgtttcc ccctggaagc tccctcgtgc 2580
   gctctcctgt tccgaccctg ccgcttaccg gatacctgtc cgcctttctc ccttcgggaa 2640
   gcgtggcgct ttctcatagc tcacgctgta ggtatctcag ttcggtgtag gtcgttcgct 2700
   ccaagctggg ctgtgtgcac gaaccccccg ttcagcccga ccgctgcgcc ttatccggta 2760
   actatcgtct tgagtccaac ccggtaagac acgacttatc gccactggca gcagccactg 2820
   gtaacaggat tagcagagcg aggtatgtag gcggtgctac agagttcttg aagtggtggc 2880
   ctaactacgg ctacactaga aggacagtat ttggtatctg cgctctgctg aagccagtta 2940
   ccttcggaaa aagagttggt agctcttgat ccggcaaaca aaccaccgct ggtagcggtg 3000
   gtttttttgt ttgcaagcag cagattacgc gcagaaaaaa aggatctcaa gaagatcctt 3060
   tgatcttttc tacggggtct gacgctcagt ggaacgaaaa ctcacgttaa gggattttgg    3120
   tcatgagatt atcaaaaagg atcttcacct agatcctttt aaattaaaaa tgaagtttta    3180
   aatcaatcta aagtatatat gagtaaactt ggtctgacag ttaccaatgc ttaatcagtg    3240
   aggcacctat ctcagcgatc tgtctatttc gttcatccat agttgcctga ctccccgtcg    3300
   tgtagataac tacgatacgg gagggcttac catctggccc cagtgctgca atgataccgc    3360
   gagacccacg ctcaccggct ccagatttat cagcaataaa ccagccagcc ggaagggccg    3420
   agcgcagaag tggtcctgca actttatccg cctccatcca gtctattaat tgttgccggg    3480
   aagctagagt aagtagttcg ccagttaata gtttgcgcaa cgttgttgcc attgctacag    3540
   gcatcgtggt gtcacgctcg tcgtttggta tggcttcatt cagctccggt tcccaacgat    3600
   caaggcgagt tacatgatcc cccatgttgt gcaaaaaagc ggttagctcc ttcggtcctc    3660
   cgatcgttgt cagaagtaag ttggccgcag tgttatcact catggttatg gcagcactgc    3720
   ataattctct tactgtcatg ccatccgtaa gatgcttttc tgtgactggt gagtactcaa    3780
   ccaagtcatt ctgagaatag tgtatgcggc gaccgagttg ctcttgcccg gcgtcaatac    3840
   gggataatac cgcgccacat agcagaactt taaaagtgct catcattgga aaacgttctt    3900
   cggggcgaaa actctcaagg atcttaccgc tgttgagatc cagttcgatg taacccactc    3960
   gtgcacccaa ctgatcttca gcatctttta ctttcaccag cgtttctggg tgagcaaaaa    4020
   caggaaggca aaatgccgca aaaaagggaa taagggcgac acggaaatgt tgaatactca    4080
   tactcttcct ttttcaatat tattgaagca tttatcaggg ttattgtctc atgagcggat    4140
   acatatttga atgtatttag aaaaataaac aaataggggt tccgcgcaca tttccccgaa    4200
   aagtgccacc tgacgtctaa gaaaccatta ttatcatgac attaacctat aaaaataggc    4260
   gtatcacgag gccctttcgt cttcac                                          4286
<210> 21
   <211> 4142
   <212> DNA
   <213> Artificial
<220>
   <223> DNA sequence of plasmid pQE60-Rv2275C
<400> 21
   ctcgagaaat cataaaaaat ttatttgctt tgtgagcgga taacaattat aatagattca 60
   attgtgagcg gataacaatt tcacacagaa ttcattaaag aggagaaatt aaccatggag 120
   ctaggcaggc gcattccgga agccaccgcc caggaagggt ttctggttcg gccattcacc 180
   caacaatgtc agatcatcca caccgaagga gatcatgctg ttatcggggt atccccgggg 240
   aacagttact tctcccgcca gcgcctacgg gatctcgggc tttggggtct cacgaatttt 300
   gatcgtgtgg acttcgtcta caccgatgtc catgtcgccg agagttacga agcgctaggc 360
   gattccgcaa tcgaagcccg gcgcaaggcg gtcaaaaaca tccgcggcgt ccgcgccaag 420
   atcaccacca cggtgaacga actcgatccg gccggggccc ggctgtgcgt tcgtccgatg 480
   tcggagttcc agtccaacga ggcataccgg gagctgcatg cggacctgct cacgcgcctg    540
   aaagacgacg aggacttgcg cgccgtctgc caggacctag tgcggcgctt cctgtccacg    600
   aaagtgggtc cgcggcaggg ggcgacggct actcaagagc aggtgtgcat ggactacatt    660
   tgcgccgagg ccccgctatt cctcgacaca cctgcgattc tcggagtgcc gtcgtcgttg    720
   aattgctacc accaatcact gcccctcgcc gaaatgctct acgcccgagg atcgggacta    780
   cgggcatcgc gcaatcaagg ccacgccatt gttacccctg atgggagccc cgccgaaaga    840
   tctcatcacc atcaccatca ctaagcttaa ttagctgagc ttggactcct gttgatagat    900
   ccagtaatga cctcagaact ccatctggat ttgttcagaa cgctcggttg ccgccgggcg    960
   tttttattgg gtgagaatcc aagctagctt ggcgagattt tcaggagcta aggaagctaa   1020
   aatggagaaa aaaatcactg gatataccac cgttgatata tcccaatggc atcgtaaaga   1080
   acattttgag gcatttcagt cagttgctca atgtacctat aaccagaccg ttcagctgga   1140
   tattacggcc tttttaaaga ccgtaaagaa aaataagcac aagttttatc cggcctttat   1200
   tcacattctt gcccgcctga tgaatgctca tccggaattt cgtatggcaa tgaaagacgg   1260
   tgagctggtg atatgggata gtgttcaccc ttgttacacc gttttccatg agcaaactga   1320
   aacgttttca tcgctctgga gtgaatacca cgacgatttc cggcagtttc tacacatata   1380
   ttcgcaagat gtggcgtgtt acggtgaaaa cctggcctat ttccctaaag ggtttattga   1440
   gaatatgttt ttcgtctcag ccaatccctg ggtgagtttc accagttttg atttaaacgt   1500
   ggccaatatg gacaacttct tcgcccccgt tttcaccatg catgggcaaa tattatacgc   1560
   aaggcgacaa ggtgctgatg ccgctggcga ttcaggttca tcatgccgtc tgtgatggct   1620
   tccatgtcgg cagaatgctt aatgaattac aacagtactg cgatgagtgg cagggcgggg   1680
   cgtaattttt ttaaggcagt tattggtgcc cttaaacgcc tggggtaatg actctctagc   1740
   ttgaggcatc aaataaaacg aaaggctcag tcgaaagact gggcctttcg ttttatctgt   1800
   tgtttgtcgg tgaacgctct cctgagtagg acaaatccgc cgctctagag ctgcctcgcg   1860
   cgtttcggtg atgacggtga aaacctctga cacatgcagc tcccggagac ggtcacagct   1920
   tgtctgtaag cggatgccgg gagcagacaa gcccgtcagg gcgcgtcagc gggtgttggc   1980
   gggtgtcggg gcgcagccat gacccagtca cgtagcgata gcggagtgta tactggctta   2040
   actatgcggc atcagagcag attgtactga gagtgcacca tatgcggtgt gaaataccgc   2100
   acagatgcgt aaggagaaaa taccgcatca ggcgctcttc cgcttcctcg ctcactgact   2160
   cgctgcgctc ggtcgttcgg ctgcggcgag cggtatcagc tcactcaaag gcggtaatac   2220
   ggttatccac agaatcaggg gataacgcag gaaagaacat gtgagcaaaa ggccagcaaa   2280
   aggccaggaa ccgtaaaaag gccgcgttgc tggcgttttt ccataggctc cgcccccctg   2340
   acgagcatca caaaaatcga cgctcaagtc agaggtggcg aaacccgaca ggactataaa   2400
   gataccaggc gtttccccct ggaagctccc tcgtgcgctc tcctgttccg accctgccgc   2460
   ttaccggata cctgtccgcc tttctccctt cgggaagcgt ggcgctttct catagctcac   2520
   gctgtaggta tctcagttcg gtgtaggtcg ttcgctccaa gctgggctgt gtgcacgaac   2580
   cccccgttca gcccgaccgc tgcgccttat ccggtaacta tcgtcttgag tccaacccgg   2640
   taagacacga cttatcgcca ctggcagcag ccactggtaa caggattagc agagcgaggt   2700
   atgtaggcgg tgctacagag ttcttgaagt ggtggcctaa ctacggctac actagaagga   2760
   cagtatttgg tatctgcgct ctgctgaagc cagttacctt cggaaaaaga gttggtagct   2820
   cttgatccgg caaacaaacc accgctggta gcggtggttt ttttgtttgc aagcagcaga   2880
   ttacgcgcag aaaaaaagga tctcaagaag atcctttgat cttttctacg gggtctgacg   2940
   ctcagtggaa cgaaaactca cgttaaggga ttttggtcat gagattatca aaaaggatct   3000
   tcacctagat ccttttaaat taaaaatgaa gttttaaatc aatctaaagt atatatgagt   3060
   aaacttggtc tgacagttac caatgcttaa tcagtgaggc acctatctca gcgatctgtc   3120
   tatttcgttc atccatagtt gcctgactcc ccgtcgtgta gataactacg atacgggagg   3180
   gcttaccatc tggccccagt gctgcaatga taccgcgaga cccacgctca ccggctccag   3240
   atttatcagc aataaaccag ccagccggaa gggccgagcg cagaagtggt cctgcaactt   3300
   tatccgcctc catccagtct attaattgtt gccgggaagc tagagtaagt agttcgccag   3360
   ttaatagttt gcgcaacgtt gttgccattg ctacaggcat cgtggtgtca cgctcgtcgt   3420
   ttggtatggc ttcattcagc tccggttccc aacgatcaag gcgagttaca tgatccccca   3480
   tgttgtgcaa aaaagcggtt agctccttcg gtcctccgat cgttgtcaga agtaagttgg   3540
   ccgcagtgtt atcactcatg gttatggcag cactgcataa ttctcttact gtcatgccat   3600
   ccgtaagatg cttttctgtg actggtgagt actcaaccaa gtcattctga gaatagtgta   3660
   tgcggcgacc gagttgctct tgcccggcgt caatacggga taataccgcg ccacatagca   3720
   gaactttaaa agtgctcatc attggaaaac gttcttcggg gcgaaaactc tcaaggatct   3780
   taccgctgtt gagatccagt tcgatgtaac ccactcgtgc acccaactga tcttcagcat   3840
   cttttacttt caccagcgtt tctgggtgag caaaaacagg aaggcaaaat gccgcaaaaa   3900
   agggaataag ggcgacacgg aaatgttgaa tactcatact cttccttttt caatattatt   3960
   gaagcattta tcagggttat tgtctcatga gcggatacat atttgaatgt atttagaaaa   4020
   ataaacaaat aggggttccg cgcacatttc cccgaaaagt gccacctgac gtctaagaaa   4080
   ccattattat catgacatta acctataaaa ataggcgtat cacgaggccc tttcgtcttc   4140
   ac                                                                  4142